# EUROPEAN PATENT SPECIFICATION

(11) **EP 3 399 995 B1**
(45) Date of publication and mention of the grant of the patent: **02.12.2026**
(21) Application number: 17736434.6
(22) Date of filing: 06.01.2017
(51) Int. Cl.: A61K 38/12, A61P 31/10

(54) **A SEMI-SYNTHETIC ECHINOCANDIN DERIVATIVE FOR USE IN PREVENTING AND TREATING PNEUMOCYSTIS INFECTIONS**
SEMI-SYNTHETISCHES ECHINOCANDIN-DERIVAT ZUR VERWENDUNG BEI DER PRÄVENTION UND BEHANDLUNG VON PNEUMOCYSTIS-INFEKTIONEN
DÉRIVÉ D'ÉCHINOCANDINE SEMI-SYNTHÉTIQUE DESTINÉ À ÊTRE UTILISÉ DANS LA PRÉVENTION ET LE TRAITEMENT DES INFECTIONS À PNEUMOCYSTIS

(30) Priority: 08.01.2016 US 201662276370 P; 16.03.2016 US 201662309316 P; 15.06.2016 US 201662350577 P
(43) Date of publication of application: 14.11.2018
(73) Proprietor: Napp Pharmaceutical Group Limited, Cambridge CB4 0GW (GB)
(72) Inventor: BARTIZAL, Kenneth, La Jolla CA 92037 (US); DARUWALA, Paul, Del Mar CA 92014 (US); ONG, Voon, San Diego CA 92128 (US); RODEN, Maureen, Severna Park MD 21146 (US); SANDISON, Taylor, Encinitas CA 92024 (US); THYE, Dirk, San Diego CA 92116 (US)
(74) Representative: Carpmaels & Ransford LLP
(86) International application number: PCT/US2017/012533
(87) International publication number: WO 2017/120471

(56) References cited:
- WO-A1-95/08341
- WO-A2-2012/119065
- US-A- 5 166 135
- US-A- 6 069 126
- US-A1- 2015 087 583
- US-A1- 2016 213 742
- ANNALORO C ET AL: "Caspofungin treatment of Pneumocystis pneumonia during conditioning for bone marrow transplantation", EUROPEAN JOURNAL OF CLINICAL MICROBIOLOGY & INFECTIOUS DISEASES, SPRINGER, BERLIN, DE, vol. 25, no. 1, 1 January 2006 (2006-01-01), pages 52 - 54, XP019355449, ISSN: 1435-4373, DOI: 10.1007/S10096-005-0065-Z
- M. ELENA CEBALLOS ET AL: "Successful treatment with echinocandin in an HIV-infected individual failing first-line therapy for Pneumocystis jirovecii pneumonia :", AIDS, vol. 25, no. 17, 1 November 2011 (2011-11-01), GB, pages 2192 - 2193, XP055605615, ISSN: 0269-9370, DOI: 10.1097/QAD.0b013e32834c4c3c
- ONG V ET AL: "Preclinical Evaluation Shows CD101, a Novel Echinocandin, is Highly Stable with No Hepatotoxicity in Rats", 17 September 2015 (2015-09-17), pages 1 - 1, XP055927118, Retrieved from the Internet <URL:https://www.cidara.com/wp-content/uploads/2015/11/2015-ICAAC_CD101_Ong_Preclinical_A-015_10Sep_FINAL.pdf> [retrieved on 20220601]
- ONG V ET AL: "Prolonged Efficacy Following One Dose of a Novel Echinocandin, CD101, in a Neutropenic Mouse Model of Disseminated Candidiasis", 17 September 2015 (2015-09-17), pages 1 - 1, XP055927122, Retrieved from the Internet <URL:https://www.eurofins.com/media/313673/eurofins_cidera.pdf> [retrieved on 20220601]
- PEEDICAYIL ABRAHAM ET AL: "Risk of Ovarian Cancer and Inherited Variants in Relapse-Associated Genes", PLOS ONE, vol. 5, no. 1, 27 January 2010 (2010-01-27), US, pages e8884 - 8, XP055856745, ISSN: 1932-6203, DOI: 10.1371/journal.pone.0008884
- DATABASE PUBCHEM 8 October 2012 (2012-10-08), "Substance Record", XP055398237, Database accession no. 144216468

## Description

### BACKGROUND

*Pneumocystis* spp. are opportunistic pathogenic fungi that cause *Pneumocystis* infections (e.g., pneumocystis pneumonia (PCP)) in mammalian hosts with compromised immune systems. PCP is not responsive to standard antifungal therapy and there are few treatment alternatives. There is a need in the art for methods of preventing and treating *Pneumocystis* infections.

WO 95/08341 relates to antifungal and anti-Pneumocystis compounds with an Echinocandin B nucleus and with a polyphenylene side chain attached to the dihydroxyornithine residue at the N2 position and an aminoalkyl ether derivative at the dihydroxyornithine at the C5 position
US 2015/087583 relates to pharmaceutical compositions, methods, and kits featuring dosing regimens and oral dosage formulations for administration of echinocandin class compounds.

ANNALORO C ET AL: "Caspofungin treatment of Pneumocystis pneumonia during conditioning for bone marrow transplantation", EUROPEAN JOURNAL OF CLINICAL MICROBIOLOGY & INFECTIOUS DISEASES, SPRINGER, BERLIN, DE, vol. 25, no. 1, 1 January 2006, pages 52-54 relates to caspofungin treatment of Pneumocystis pneumonia during conditioning for bone marrow transplantation.

M. ELENA CEBALLOS ET AL: "Successful treatment with echinocandin in an HIV-infected individual failing first-line therapy for Pneumocystis jirovecii pneumonia", AIDS, vol. 25, no. 17, 1 November 2011, pages 2192-2193 relates to treatment with echinocandin in an HIV-infected individual failing first-line therapy for Pneumocystis jirovecii pneumonia.

### SUMMARY

The invention is defined by the appended claims.

In particular, a first aspect of the invention relates to a compound for use in a method for treating *a Pneumocystis* infection (e.g., an infection caused by *Pneumocystis jirovecii* in humans) or reducing the risk of a *Pneumocystis* infection in a subject (e.g., a human), the method comprising administering to the subject the compound in an amount and for a duration sufficient to treat the *Pneumocystis* infection, wherein the compound is Compound 1: 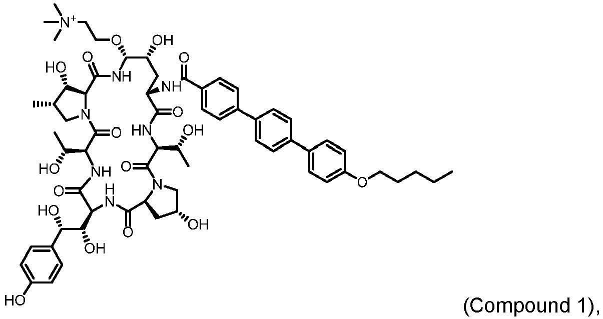 or a pharmaceutically acceptable salt thereof. In some embodiments, the subject is immunocompromised, and thus, especially vulnerable to *Pneumocystis* infections.

In some embodiments, the treating includes reducing the cyst burden in the lung of the subject. In other embodiments, the treating includes inhibiting the reproduction of a *Pneumocystis* spp. in the subject. In particular embodiments, the subject being treated has *Pneumocystis* pneumonia.

In any of the above embodiments, the compound can be administered intraorally, intravenously, intramuscularly, intradermally, intraarterially, subcutaneously, orally, or by inhalation. In some embodiments, the compound is administered intravenously. In some embodiments, the compound is administered by inhalation. In some embodiments, the compound is administered orally. In certain embodiments, the subject is a human and the *Pneumocystis* infection is caused by *Pneumocystis jirovecii.*

For example, the compound of the invention can be for use in treating or reducing the risk of a *Pneumocystis* infection in a subject that is HIV positive or has hyper IgM syndrome. In one particular embodiment, the immunocompromised subject has a CD4⁺ T-cell count of less than 200 cells/µl of blood. In still other embodiments, the compound or pharmaceutically acceptable salt thereof, is administered concurrently or within 7 days, 5 days, 3 days, or 1 day, before and/or after, the subject undergoes immunosuppression therapy. For example, the compound or pharmaceutically acceptable salt thereof can be administered concurrently or within 7 days, 5 days, 3 days, or 1 day, before and/or after, the subject undergoes an anti-TNF therapy, a corticosteroid therapy, a chemotherapy, or any other treatment regimen or procedure associated with, or accompanied by, a suppressed immune system. In particular embodiments, the compound or pharmaceutically acceptable salt thereof is administered concurrently or within 7 days, 5 days, 3 days, or 1 day, before and/or after, the subject undergoes a transplantation procedure. The transplantation procedure can be an organ transplant (e.g., a solid organ transplant) or a bone marrow transplant.

In any of the above embodiments, the compound or a pharmaceutically acceptable salt thereof can be administered concurrently with a second antifungal agent. In some embodiments, the second antifungal agent is clindamycin, trimethoprim, sulfamethoxazole, cotrimoxazole, atovaquone, pentamidine, primaquine, pyrimethamine, or a pharmaceutically acceptable salt thereof. In other embodiments, the second antifungal agent is selected from glucan synthase inhibitors, polyene compounds, azole compounds, and pharmaceutically acceptable salts thereof. For example, the second antifungal agent can be any glucan synthase inhibitor, polyene compound, or azole compound described herein.

A second aspect of the invention provides a compound for use in an *in vivo* method of inhibiting the replication of a *Pneumocystis* spp., wherein the method includes contacting the *Pneumocystis* spp. with the compound in an amount and for a duration sufficient to inhibit replication of the *Pneumocystis* spp., wherein the compound is Compound 1: 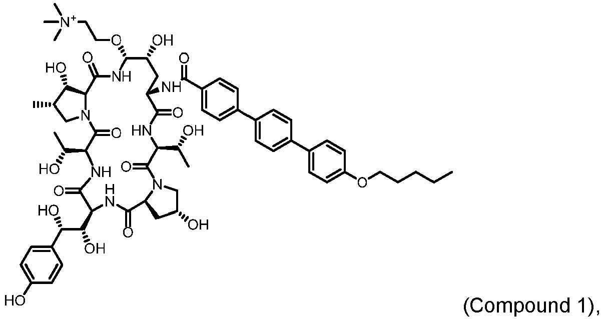 or a pharmaceutically acceptable salt thereof. In some embodiments, the *Pneumocystis* spp. is *Pneumocystis jirovecii.* Particular embodiments described herein include administering to the subject doses of about 150 mg to about 800 mg of a salt of Compound 1, or a neutral form thereof, one to three times per week to the subject for a period of 2 to 8 weeks.

In some embodiments of the invention, the method includes weekly dosing of Compound 1 in salt or neutral form, and includes (a) intravenously administering a first dose of about 400 mg of Compound 1 in salt or neutral form, (b) intravenously administering a subsequent dose of about 200 mg of Compound 1 in salt or neutral form, and (c) optionally repeating step (b) for one to ten weeks.

In some embodiments of the invention, the method consists of (a) intravenously administering a first dose of about 400 mg of Compound 1 in salt or neutral form, (b) intravenously administering a second dose of 200 mg of Compound 1 in salt or neutral form, and (c) optionally intravenously administering a third dose of about 200 mg of Compound 1 in salt or neutral form, wherein the first dose is administered on day 1, the second dose is administered on day 8, and the third dose, if administered, is administered on day 15.

In some embodiments of the invention, the method includes weekly dosing of Compound 1 in salt or neutral form, and includes (a) intravenously administering a dose of about 400 mg of Compound 1 in salt or neutral form, (b) intravenously administering a subsequent dose of about 400 mg of Compound 1 in salt or neutral form, and (c) optionally repeating step (b) for one to ten weeks.

In some embodiments of the invention, the method consists of (a) intravenously administering a first dose of about 400 mg of Compound 1 in salt or neutral form, (b) intravenously administering a second dose of about 400 mg of Compound 1 in salt or neutral form, and (c) optionally intravenously administering a third dose of about 400 mg of Compound 1 in salt or neutral form, wherein the first dose is administered on day 1, the second dose is administered on day 8, and the third dose, if administered, is administered on day 15.

In some embodiments, the third dose of about 200 mg of Compound 1 in salt or neutral form is administered if on day 15 mycological eradication and/or clinical cure has not been achieved in the subject. In other embodiments, the third dose including 200 mg of Compound 1 in salt or neutral form is not administered if mycological eradication has been achieved in the subject.

In some embodiments, the third dose of about 400 mg of Compound 1 in salt or neutral form is administered if on day 15 mycological eradication and/or clinical cure has not been achieved in the subject. In other embodiments, the third dose including 400 mg of Compound 1 in salt or neutral form is not administered if mycological eradication has been achieved in the subject.

In some embodiments, mycological eradication is determined by two negative blood cultures drawn at ≥ 12 hours apart without intervening positive blood cultures.

In some embodiments, the third dose of about 200 mg of Compound 1 in salt or neutral form is administered if on day 15 the subject displays symptoms of a fungal infection. In some embodiments, the third dose of about 400 mg of Compound 1 in salt or neutral form is administered if on day 15 the subject displays symptoms of a fungal infection. In some embodiments, symptoms of the fungal infection includes fever, cough, shortness of breath, weight loss, and/or night sweats.

In some embodiments, Compound 1 is administered over a time period of 30 to 180 minutes (e.g., over 30±5 minutes, 60±5 minutes, 90±5 minutes, 120±5 minutes, 150±5 minutes, or 180±5 minutes). In some embodiments, Compound 1 is administered as an aqueous pharmaceutical composition. In some embodiments, the pharmaceutical composition has a pH of from 4.0 to 8. In some embodiments, Compound 1 salt is Compound 1 acetate.

As used herein, the amount in each dose refers to the amount of Compound 1 (structure shown above) that does not include the negative counterion (e.g., an acetate) if Compound 1 is in its salt form. For example, a dose of about 400 mg or 200 mg of Compound 1 in salt or neutral form refers to 400 mg or 200 mg of Compound 1, not including the acetate ion if Compound 1 is in an acetate salt form.

In some embodiments, Compound 1 in salt or neutral form is administered for a predetermined period of 2-12 doses (e.g., 2-3 doses). In some embodiments, Compound 1 in salt or neutral form is administered until mycological eradication as determined by a standard test known in the art. In some embodiments, mycological eradication is defined as two negative blood cultures drawn at ≥ 12 hours apart without intervening positive blood cultures and no change of antifungal therapy for the fungal infection (e.g., a *Pneumocystis* infection). In some embodiments, Compound 1 in salt or neutral form is administered until the subject is free of symptoms of the fungal infection (e.g., a *Pneumocystis* infection), such as fever, cough, shortness of breath, weight loss, and night sweats, as determined by a physician.

In some embodiments of the compound of the invention for use in methods of reducing the risk of *a Pneumocystis* infection in a subject, more than 3 doses (e.g., 4, 5, 6, 7, 8, 9, or 10 doses) of Compound 1 in salt or neutral form is administered to the subject. In some embodiments, the first dose includes about 400 mg of Compound 1 in salt or neutral form, and the remaining doses each includes about 200 mg of Compound 1 in salt or neutral form. In some embodiments, all doses each includes about 400 mg of Compound 1 in salt or neutral form.

### Definitions

As used herein, the term *"Pneumocystis* infection" refers to a fungal infection caused by a fungus in the genus *Pneumocystis,* e.g., *P. carinii, P. jirovecii, P. murina, P. oryctolagi,* and *P*. *wakefieldiae.*

As used herein, the term "reducing the risk of a *Pneumocystis* infection" refers to reducing the likelihood of a subject (e.g., a human) getting a *Pneumocystis* infection. The subject receiving prophylactic therapy can be a subject in need of such treatment, such as an immunocompromised subject.

As used herein, the term "inhibiting the reproduction or growth of a *Pneumocystis* spp." refers to reducing or preventing the reproduction of a *Pneumocystis* spp., reducing or preventing the formation or growth of the the cyst form of a *Pneumocystis* spp. and/or and reducing or preventing the formation of the trophic form of a *Pneumocystis* spp. In some embodiments, the term refers to reducing the formation of the cyst form of a *Pneumocystis* spp. or preventing the formation of the cyst form of a *Pneumocystis* spp. In some embodiments, the term refers to reducing the growth of the cyst form of a *Pneumocystis* spp. or preventing the growth of the cyst form of a *Pneumocystis* spp. In some embodiments, the term refers to reducing the formation of the trophic form of a *Pneumocystis* spp. or preventing the formation of the trophic form of a *Pneumocystis* spp. In some embodiments, the term refers to reducing the growth of the trophic form of a *Pneumocystis* spp. or preventing the growth of the trophic form of a *Pneumocystis* spp. As currently understood, and without being bound by theory, *Pneumocystis* spp. exist within a host in two forms: the trophic and the cyst. The trophic forms of *Pneumocystis* spp. may reproduce asexually via binary fission while cysts are able to reproduce sexually after trophozoites conjugate. The cysts develop in three stages with increasing amounts of intracystic nuclei in each stage. The early cyst stage contains two intracystic nuclei while the intermediate stage has four and the late precysts stage holds eight. The mature cysts then release the trophic form of *Pneumocystis* spp. as they burst. The compounds described herein can be useful for methods of inhibiting the formation or growth of the cyst form of *Pneumocystis* spp., and inhibiting the production of the trophic form of a *Pneumocystis* spp.

As used herein, the term "cyst burden" refers to the amount (i.e., number) of cysts present *in vivo* as measured in tissues of an infected subject, or *in vitro* as measured in a sample (i.e., a tissue sample) isolated from an infected subject. A cyst refers to a group of encased spores that form the trophic form of *a Pneumocystis* spp. after release from the cyst.

As used herein, the term *"Pneumocystis* pneumonia" refers to an infection caused by *Pneumocystis* spp. (e.g., in humans *Pneumocystis jirovecii*)*.* Depending upon the mammal infected, *Pneumocystis* pneumonia may also be referred to as *Pneumocystis jirovecii* pneumonia, *Pneumocystis carinii* pneumonia, or PCP.

As used herein, the term "immunocompromised" refers to a subject (e.g., a human) having a weakened immune system. The subject's immune system can be weakened or compromised by a disease (e.g., an HIV infection, an autoimmune disease, cancer), a medical procedure (e.g., an organ transplant (e.g., a solid organ transplant) or a bone marrow transplant), a drug (e.g., an immunosuppressant), and/or a pathogen (e.g., bacteria, fungus, virus). The immune system of the host may also have a congenital defect that renders the host more susceptible to infection.

As used herein, the term "immunosuppression therapy" refers to a therapy that uses one or more immunosuppressants to reduce the activation and/or efficacy of the immune system of a subject (e.g., a human). In some embodiments, an immunosuppression therapy is used to prevent the body from rejecting a transplant (e.g., an organ transplant (e.g., a solid organ transplant) or a bone marrow transplant), to treat graft-versus-host disease after a bone marrow transplant, and/or to treat autoimmune diseases (e.g., systemic lupus erythematosus, rheumatoid arthritis, Crohn's disease, multiple sclerosis, myasthenia gravis, Sarcoidosis, Behcet's disease). Examples of immunosuppressants include, but are not limited to, calcineurin inhibitors, mTOR inhibitors, and tyrosine kinase inhibitors (e.g., cyclosporine A, cyclosporine G, voclosporin, tacrolimus, pimecrolimus, sirolimus, temsirolimus, deforolimus, everolimus, zotarolimus, biolimus, imatinib, dasatinib, nilotinib, erlotinib, sunitinib, gefitinib, bosutinib, neratinib, axitinib, crizotinib, lapatinib, toceranib and vatalanib).

As used herein, the term "anti-TNF therapy" refers to a therapy that uses small molecule and/or protein drugs to inhibit or prevent tumor necrosis factor (TNF) receptor binding and/or activation by a TNF. TNF signaling is involved in the autoimmune and immune-mediated disorders (e.g., systemic lupus erythematosus, rheumatoid arthritis, Crohn's disease, multiple sclerosis, myasthenia gravis, Sarcoidosis, Behcet's disease). Examples of small molecule and/or protein drugs that target the TNF receptor and/or the TNF include, but are not limited to, infliximab, adalimumab, certolizumab pegol, etanercept, golimumab, xanthine derivatives, and bupropion.

As used herein, the term "corticosteroid therapy" refers to a therapy that uses one or more corticosteroids to treat a variety of diseases and conditions, e.g., immune system-related diseases, inflammatory conditions, and skin diseases. Examples of corticosteroids include, but are not limited to, dexamethasone, prednisone, fludrocortisones, and hydrocortisone.

As used herein, the term "chemotherapy" refers to a cancer treatment that uses one or more anticancer drugs. In cancer treatment, chemotherapy is sometimes used in combination with other therapies, such as radiation and surgery to treat cancer.

As used herein, the term "second antifungal agent" refers to an agent that is used in combination with a compound of any of formulas (I)-(III) (e.g., a salt form of Compound 1) to treat a fungal infection (e.g., a *Pneumocystis* infection). Examples of second antifungal agents include, but are not limited to, any of the antifungal agents described herein.

As used herein, the term "a neutral form" includes zwitterionic forms of compounds bearing a tertiary ammonium ion positive charge (e.g., Compound 1 or Compound 2) in which the compound has no net positive or negative charge. The zwitterion can be present in a higher proportion in basic medium (e.g., pH 9).

As used herein, the terms "intravenous administration" or "intravenously administering" refer to intravenous bolus injection or infusion of a drug to a subject (e.g., a human).

As used herein, the term "about" refers to a range of values that is ± 10% of specific value. For example, "about 150 mg" includes ± 10% of 150 mg, or from 135 mg to 165 mg. Such a range performs the desired function or achieves the desired result. For example, "about" may refer to an amount that is within less than 10% of, within less than 5% of, within less than 1% of, within less than 0.1% of, and within less than 0.01% of the stated amount.

By "dose" is meant the amount of Compound 1 administered to the subject (e.g., a human).

By "clinical cure" is meant complete resolution of most or all of the clinical signs and symptoms of candidemia which were present at baseline and no new signs/symptoms or complications attributable to candidemia.

### DESCRIPTION OF THE DRAWINGS

FIG. 1A shows the preventative effects of Compound 1 on lung burdens (shown as nuclei counts) in mice infected with *Pneumocystis murina.*
FIG. 1B shows the preventative effects of Compound 1 on lung burdens (shown as asci counts) in mice infected with *Pneumocystis murina.*
FIG. 2A shows the therapeutic effects of Compound 1 on lung burdens (shown as nuclei counts) in mice infected with *Pneumocystis murina.*
FIG. 2B shows the therapeutic effects of Compound 1 on lung burdens (shown as asci counts) in mice infected with *Pneumocystis murina.*
FIG. 3 shows the structures of several reference compounds: Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, and Compound 19.
FIG. 4 shows PK of Compound 1 over doses 1 mg/kg, 4 mg/kg, and 16 mg/kg.
FIG. 5 shows net change in fungal density (log₁₀ CFU) versus different total doses of Compound 1 at different fractionation schedules.
FIG. 6 shows change in fungal density (log₁₀ CFU) reduction from baseline caused by 2 mg/kg total dose of Compound 1 at different fractionation schedules.
FIG. 7 shows simulated free-drug concentration time profiles relative to the MIC for the fractionated Compound 1 2 mg/kg regimen.

### DETAILED DESCRIPTION

The invention is defined by the appended claims. Provided is Compound 1 or a pharmaceutically acceptable salt thereof, for use in methods for treating or reducing the risk of a *Pneumocystis* infection (e.g., a *Pneumocystis jirovecii* infection in a human) in a subject (e.g., a human) by administering the compound to the subject. In some embodiments, the subject is immunocompromised and, thus, especially vulnerable to *Pneumocystis* infection. The invention also provides Compound 1 or a pharmaceutically acceptable salt thereof, for use in methods of inhibiting the replication of a *Pneumocystis* spp.

Any references in the description to methods of treatment refer to the compound of the invention for use in the corresponding method for treatment of the human (or animal) body.

### I. Pneumocystis Infection

*A Pneumocystis* infection refers to an infection caused by a fungus in the genus *Pneumocystis.* Fungi in the genus *Pneumocystis* include P. *carinii, P. jirovecii, P. murina, P. oryctolagi,* and P. *wakefieldiae. Pneumocystis jirovecii* pneumonia (also called *Pneumocystis carinii* pneumonia, *Pneumocystis* pneumonia, or PCP) caused by *Pneumocystis jirovecii* remains an important cause of morbidity and mortality in immunocompromised human subjects. PCP is not commonly found in the lungs of healthy subjects, but it can cause lung infections in subjects with weak immune systems. Within the alveolar lumen, *Pneumocystis* spp. appear to have a bi-phasic life cycle consisting of an asexual phase characterized by binary fission of trophic forms and a sexual cycle resulting in formation of cysts. The cysts express 1,3-β-D-glucan synthase and contain 1,3-β-D-glucan. Some signs and symptoms of PCP include, but are not limited to, fever, cough, shortness of breath, weight loss, and night sweats. In certain cases, the fungi can invade other organs, such as the brain, liver, spleen, and kidney. Pneumothorax is often a complication of PCP. Diagnosis of PCP can be confirmed by techniques and methods well-known in the art, e.g., chest x-ray and/or gallium 67 scans. The diagnosis can also be confirmed by histological identification of the causative organism in sputum or bronchio-alveolar lavage. Staining with, e.g., toluidine blue, silver stain, or periodic-acid Schiff stain, or an immunofluorescence assay often shows the characteristic cysts and/or trophic forms.

There is a significant need for prophylaxis against PCP, especially in immunocompromised subjects. However, routine, continuous cultivation of *Pneumocystis in vitro* for testing of susceptibility to antimicrobial agents has not been achieved. Immunosuppressed-rodent models of *Pneumocystis* infection have been highly predictive of the clinical efficacy of standard of care *anti-Pneumocystis* therapies, e.g., trimethoprim-sulfamethoxazole (TMP-SMX) and dapsone. TMP-SMX targets dihydropteroate synthase (DHPS) and dihydrofolate reductase (DHFR). However, up to one-third of subjects with PCP are intolerant of or fail to respond to treatment with TMP-SMX. Toxicity associated with the administration of TMP-SMX include, e.g., fever, rash, headache, nausea, vomiting, neutropenia, pancytopenia, meningitis, nephrotoxicity, anaphylaxis, hepatitis, hyperkalemia, and hypoglycemia. Dapsone is an inhibitor of DHPS. Administration of dapsone also causes adverse reactions, e.g., agranulocytosis, aplastic anemia, rash, nausea, malaise, and a sulfone syndrome (fever, rash, hepatitis, lymphadenopathy, and methemoglobinemia).

Most subjects with PCP have had some identifiable defect in T-cell immunity. PCP has been associated with a broad array of immune deficits. Examples of immune deficits include, but are not limited to, those caused by a human immunodeficiency virus (HIV) infection, a hyper IgM syndrome, cancers, autoimmune diseases, neutropenia, CD4 lymphopenia, hematologic disorders, a congenital immune deficiency, Cushing's syndrome, a nephrotic syndrome, an organ transplant (e.g., a solid organ transplant), a bone marrow transplant, a radiation therapy, one or more immunosuppressants (e.g., corticosteroids), an immunosuppressive therapy, an anti-TNF therapy, a corticosteroid therapy, and/or a chemotherapy. As described in detail further herein, an immunocompromised subject is especially vulnerable to a *Pneumocystis* infection (e.g., a *Pneumocystis jirovecii* infection or PCP).

### II. Immunocompromised Subjects

Immunocompromised subjects (e.g., a human) are especially vulnerable to *Pneumocystis* infections. An immunocompromised subject has an immune system that is weakened by a disease or disorder, a medical procedure (e.g., a transplantation procedure or a surgery), a drug (e.g., an immunosuppressive agent), and/or a pathogen (e.g., bacteria, fungus, virus). In some embodiments, a subject's immune system is compromised by the treatment of any of the diseases or disorders described further herein. In some embodiments, a subject (e.g., an immunocompromised subject) is about to have, is currently having, or has had a disease or disorder that weakens the immune system. In some embodiments, a subject (e.g., an immunocompromised subject) is about to undergo or has undergone a transplantation procedure (e.g., an organ transplant (e.g., a solid organ transplant) or a bone marrow transplant) or a radiation therapy. In some embodiments, a subject (e.g., an immunocompromised subject) is about to be administered, is currently administered, or has been administered one or more drugs that weaken the immune system, e.g., an immunosuppressant (e.g., a corticosteroid). In some embodiments, a subject (e.g., an immunocompromised subject) is about to undergo, is currently undergoing, or has undergone an immunosuppressive therapy, an anti-TNF therapy, a corticosteroid therapy, and/or a chemotherapy. In some embodiments, a subject's immune system is compromised by a pathogen (e.g., bacteria, fungus, virus), i.e., either the pathogen is currently present within the subject or had previously infected the subject.

In some embodiments, a subject (e.g., an immunocompromised subject) is HIV positive or has hyper IgM syndrome. In some embodiments, a subject (e.g., an immunocompromised subject) has a CD4⁺ T-cell count of less than 200 cells/µl of blood. In some embodiments, methods described herein may be used to reduce the risk of or to treat a *Pneumocystis* infection (e.g., a *Pneumocystis jirovecii* infection or PCP) in a subject who is HIV positive or has hyper IgM syndrome. In some embodiments, methods described herein may be used to reduce the risk of or to treat a *Pneumocystis* infection (e.g., a *Pneumocystis jirovecii* infection or PCP) in a subject having a CD4⁺ T-cell count of less than 200 cells/µl of blood.

Examples of diseases and disorders that weaken a subject's immune system include, but are not limited to, a human immunodeficiency virus (HIV) infection, a hyper IgM syndrome, cancers, autoimmune diseases, neutropenia, CD4 lymphopenia, hematologic disorders, a congenital immune deficiency, Cushing's syndrome, and a nephrotic syndrome. Examples of cancers that weaken a subject's immune system include, but are not limited to, leukemia (e.g., acute lymphoblastic leukemia (ALL), acute myeloid leukemia (AML), chronic myeloid leukemia (CML), small lymphocytic lymphoma (SLL), chronic lymphocytic leukemia (CLL), acute monocytic leukemia(AMOL), and erythroleukemia), Hodgkin's lymphoma, and non-Hodgkin's lymphoma (e.g., lymphoblastic lymphoma, small cell lymphoma (Burkitt's/Non-Burkitt's), and large cell lymphoma). Other cancers that may weaken a subject's immune system include, but are not limited to, bladder cancer, pancreatic cancer, lung cancer, liver cancer, ovarian cancer, colon cancer, stomach cancer, breast cancer, prostate cancer, renal cancer, testicular cancer, thyroid cancer, uterine cancer, rectal cancer, a cancer of the respiratory system, a cancer of the urinary system, oral cavity cancer, skin cancer, larynx cancer, sarcoma, carcinoma, basal cell carcinoma, choriocarcinoma, adenocarcinoma, giant (or oat) cell carcinoma, breast carcinoma, squamous cell carcinoma, multiple myeloma (MM), astrocytoma, oligoastrocytoma, biliary tract cancer, CNS cancer, neuroblastoma, glioblastoma, rhabdomyosarcoma, neuroectodermal cancer, melanoma, inflammatory myofibroblastic tumor and soft tissue tumor. Subjects having a disease or disorder that weaken a subject's immune system can reduce their risk of developing a *Pneumocystis* infection using the methods disclosed herein.

Examples of autoimmune diseases that weaken a subject's immune system include, but are not limited to, alopecia areata, ankylosing spondylitis, antiphospholipid syndrome, Addison's disease, hemolytic anemia, autoimmune hepatitis, hepatitis, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatricial pemphigoid, limited scleroderma (CREST Syndrome), cold agglutinin disease, Crohn's disease, discoid lupus, essential mixed cryoglobulinemia, fibromyalgia-fibromyositis, Graves' disease, Guillain-Barré syndrome, Hashimoto's thyroiditis, hypothyroidism, inflammatory bowel disease, autoimmune lymphoproliferative syndrome (ALPS), idiopathic pulmonary fibrosis, idiopathic thrombocytopenia purpura (ITP), IgA nephropathy, insulin-dependent diabetes, juvenile arthritis, Lichen planus, lupus, Ménière's disease, mixed connective tissue disease, multiple sclerosis, myasthenia gravis, pemphigus vulgaris, pernicious anemia, polyarteritis nodosa, polychondritis, polyglandular syndromes, polymyalgia rheumatica, polymyositis, dermatomyositis, primary agammaglobulinemia, primary biliary cirrhosis, psoriasis, Raynaud's phenomenon, Reiter's syndrome, rheumatic fever, rheumatoid arthritis, sarcoidosis, scleroderma, Sjögren's syndrome, Stiff-man syndrome, systemic lupus erythematosus, Takayasu arteritis, temporal arteritis/giant cell arteritis, ulcerative colitis, uveitis, vasculitis, vitiligo, and Wegener's granulomatosis. Subjects having an autoimmune disease can reduce their risk of developing a *Pneumocystis* infection using the methods disclosed herein.

The methods described herein can be used to reduce the risk of, or treat, a *Pneumocystis* infection (e.g., a *Pneumocystis jirovecii* infection or PCP) in a subject (e.g., a human) having an HIV infection. PCP is the most common opportunistic infection in subjects with HIV. HIV infects the cells of the immune system by attaching and destroying T-cells (e.g., CD4⁺ T-cells). An HIV-positive subject with low CD4⁺ T-cell count is especially susceptible to *Pneumocystis* infections (e.g., *Pneumocystis jirovecii* infections). When an HIV-positive subject's T-cell count falls below 200 cells/µl of blood, the subject may be susceptible to other diseases and/or infections, e.g., microbial infections (e.g., *Pneumocystis* infections), and tuberculosis, cancer.

The methods described herein can be used to reduce the risk of, or treat, a *Pneumocystis* infection (e.g., a *Pneumocystis jirovecii* infection or PCP) in a subject (e.g., a human) having a hyper IgM syndrome. Hyper IgM syndrome refers to a family of genetic disorders in which the level of IgM antibodies is relatively high as a result of a defect in a CD4⁺ Tₕ2-cell protein (e.g., CD40 ligand). The defect in the CD4⁺ Tₕ2-cell protein leads to the inability of B-cells to produce antibodies other than IgM. Subjects with hyper IgM syndrome often have a low number of neutrophils and platelets.

The methods described herein can be used to reduce the risk of, or treat, a *Pneumocystis* infection (e.g., a *Pneumocystis jirovecii* infection or PCP) in a subject having neutropenia. Neutropenia refers to a condition characterized by an abnormally low concentration of neutrophils in the blood. Neutrophils make up the majority of circulating while blood cells and serve as the primary defense against microbial infections.

The methods described herein can be used to reduce the risk of, or treat, a *Pneumocystis* infection (e.g., a *Pneumocystis jirovecii* infection or PCP) in a subject (e.g., a human) having a congenital immune deficiency. A congenital immune deficiency refers to a group of immune deficiency disorders present at the time of birth that are caused by genetic defects. Congenital immune deficiencies may occur as a result of defects in B lymphocytes and/or T lymphocytes. Subjects having a congenital immune deficiency are particularly susceptible to infections of the lung, throat, skin, and ear.

The methods described herein can be used to reduce the risk of, or treat, a *Pneumocystis* infection (e.g., a *Pneumocystis jirovecii* infection or PCP) in a subject (e.g., a human) who has undergone or is about to undergo a transplantation procedure (e.g., an organ transplant (e.g., a solid organ transplant) or a bone marrow transplant). In some embodiments, a subject has undergone or is about to undergo a hematopoietic stem cell transplant. In some embodiments, a subject who has undergone or is about to undergo a hematopoietic stem cell transplant has a hematologic disorder (e.g., leukemia). In some embodiments, a subject who has undergone or is about to undergo a transplantation procedure is administered one or more immunosuppressants before, during, and/or after the transplantation procedure to block the adverse effects of organ or tissue rejection by the immune system, or to treat or reduce complications resulting from the transplantation (e.g., graft-versus-host disease (GVHD) and graft rejection).

The methods described herein can be used to reduce the risk of, or treat, a *Pneumocystis* infection in a subject (e.g., a human) who has undergone or is about to undergo a radiation therapy. Certain types of radiation therapy may cause damage to the immune system. For example, radiation to the underarm area, where the lymph nodes are, may cause damage to the lymph nodes and vessels. Radiation directed at pelvic bones may damage the bone marrow within the bones, thus, reducing the production of red and white blood cells.

The methods described herein can be used to reduce the risk of, or treat, a *Pneumocystis* infection in a subject (e.g., a human) who is about to be administered, is currently administered, or has been administered one or more drugs that weaken the immune system, e.g., an immunosuppressant (e.g., a corticosteroid). In some embodiments, methods described herein may be used to reduce the risk of, or treat, a *Pneumocystis* infection in a subject who is about to undergo, is currently undergoing, or has undergone an immunosuppressive therapy, an anti-TNF therapy, a corticosteroid therapy, and/or a chemotherapy. Examples of immunosuppressants used in an immunosuppressive therapy that weaken a subject's immune system include, but are not limited to, calcineurin inhibitors, mTOR inhibitor, and tyrosine kinase inhibitors (e.g., cyclosporine A, cyclosporine G, voclosporin, tacrolimus, pimecrolimus, sirolimus, temsirolimus, deforolimus, everolimus, zotarolimus, biolimus, imatinib, dasatinib, nilotinib, erlotinib, sunitinib, gefitinib, bosutinib, neratinib, axitinib, crizotinib, lapatinib, toceranib and vatalanib). Examples of small molecule and protein drugs used in an anti-TNF therapy to target the TNF receptor and/or the TNF include, but are not limited to, infliximab, adalimumab, certolizumab pegol, etanercept, golimumab, xanthine derivatives, and bupropion. Examples of corticosteroids in a corticosteroid therapy that weaken a subject's immune system include, but are not limited to, dexamethasone, prednisone, fludrocortisones, and hydrocortisone.

### III. Compounds

The invention provides Compound 1 (shown below) or a pharmaceutically acceptable salt thereof for use in the methods of therapy as defined by the appended claims.

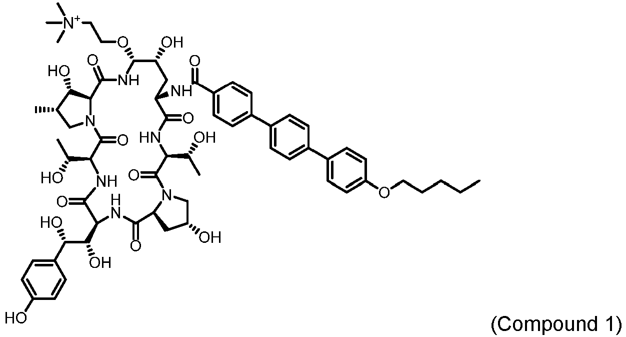

The remaining compounds of formulas (I)-(III) disclosed herein are provided for reference. The compounds of formulas (I)-(III) are shown below: 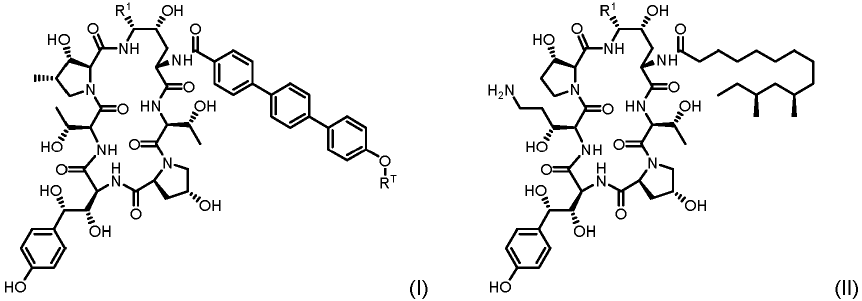 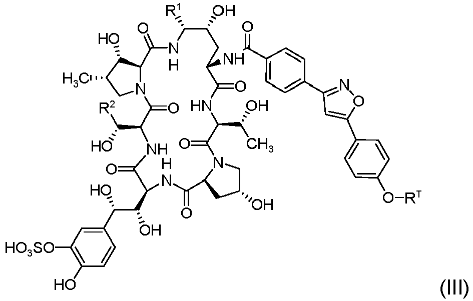 wherein R¹ is O(CH₂CH₂O)ₙCH₂CH₂X₁, O(CH₂CH₂NH)ₙCH₂CH₂X₁, O(CH₂CH₂CH₂O)ₙCH₂CH₂X₁, NHCH₂CH₂X₂, NH(CH₂CH₂O)ₘCH₂CH₂X₂, NH(CH₂CH₂CH₂O)ₘCH₂CH₂X₂, NH(CH₂CH₂O)ₚCH₂CH₂X₃, NH(CH₂CH₂CH₂O)ₚCH₂CH₂X₃, NHCH₂CH₂X₄, NH[CH₂(CH₂)ₐO]_{b}CH{CH₂[OCH₂(CH₂)_{c}]_{d}X₅)₂, O[CH₂(CH₂)ₐO]_{b}CH{CH₂[OCH₂(CH₂)_{c}]_{d}X₅}₂, NH(CH₂CH₂NH)ᵣCH₂CH₂X₅, NHCH₂(CH₂)_{q}X₆, or OCH₂(CH₂)_{q}X₆; R^{T} is n-pentyl, sec-pentyl, or iso-pentyl; X₁ is NH₂, NHR^{A1}, NR^{A1}R^{A2}, NR^{A1}R^{A2}R^{A3} or NHCH₂(CH₂)ᵥZ₁; X₂ is OH, OR^{B1}, or OCH₂(CH₂)ᵥZ₁; X₃ is NH₂, NHR^{C1}, NR^{C1}R^{C2}, or NR^{C1}R^{C2}R^{C3}, or NHCH₂(CH₂)ᵥZ₁; X₄ is NR^{D1}R^{D2}R^{D3} or NHCH₂(CH₂)ᵥZ₁; each X₅ is, independently, selected from OH, OR^{E1}, NH₂, NHR^{E1}, NR^{E1}R^{E2}, NR^{E1}R^{E2}R^{E3}, OCH₂(CH₂)ᵥZ₁, and NHCH₂(CH₂)ᵥZ₁; X₆ is selected from NR^{F1}R^{F2}R^{F3} and Z₁; a is an integer from 1 to 2; b is an integer from 0 to 3; c is an integer from 1 to 2; d is an integer from 0 to 3; n is an integer from 1 to 5; m is an integer from 1 to 5; p is an integer from 1 to 5; r is an integer from 1 to 5; q is an integer from 1 to 3; v is an integer from 1 to 3; each of R^{A1}, R^{A2}, R^{A3}, R^{B1}, R^{C1}, R^{C2}, R^{C3}, R^{D1}, R^{D2}, R^{D3}, R^{E1}, R^{E2}, R^{E3}, R^{F1}, R^{F2}, and R^{F3} is, independently, selected from CH₃, CH₂CH₃, CH₂CH₂CH₃, and CH(CH₃)₂; Z₁ is selected from: 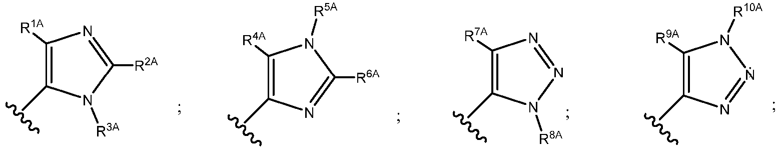 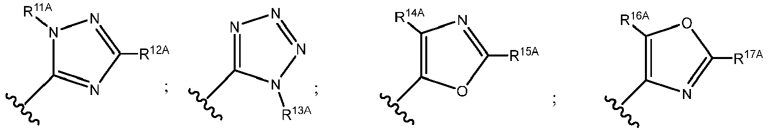 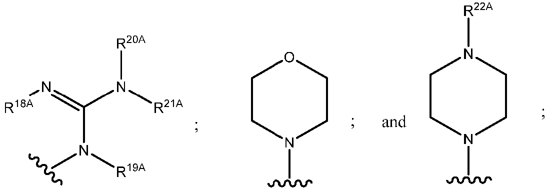 and each of R^{1A}, R^{2A}, R^{3A}, R^{4A}, R^{5A}, R^{6A}, R^{7A}, R^{8A}, R^{9A}, R^{10A}, R^{11A}, R^{12A}, R^{13A}, R^{14A}, R^{15A}, R^{16A}, R^{17A}, R^{18A}, R^{19A}, R^{20A}, R^{21A}, and R^{22A} is, independently, selected from H, CH₃, CH₂CH₃, CH₂CH₂CH₃, and CH(CH₃)₂.

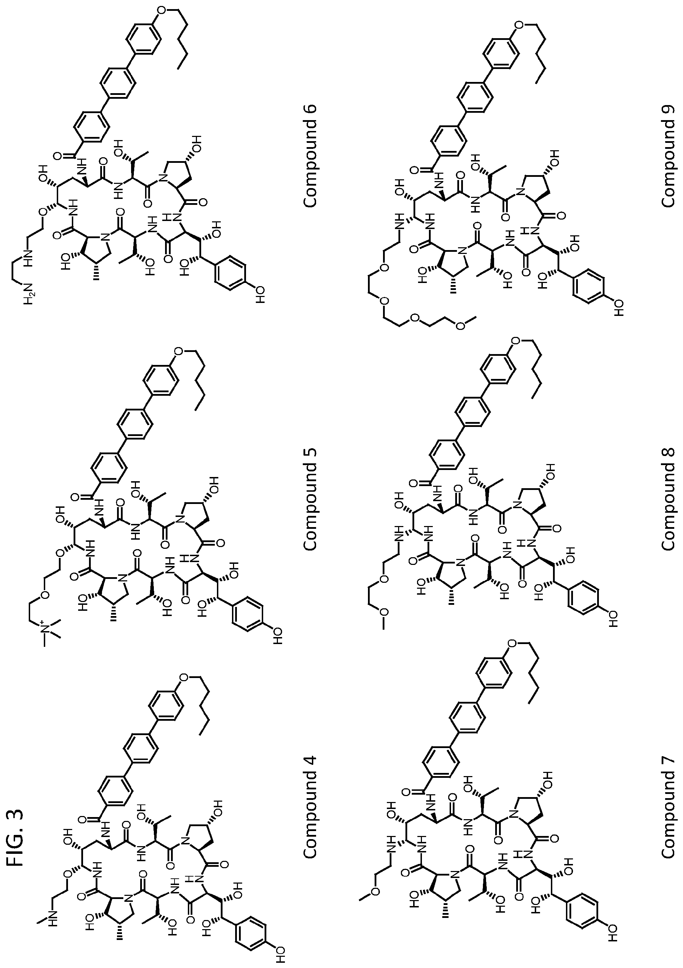

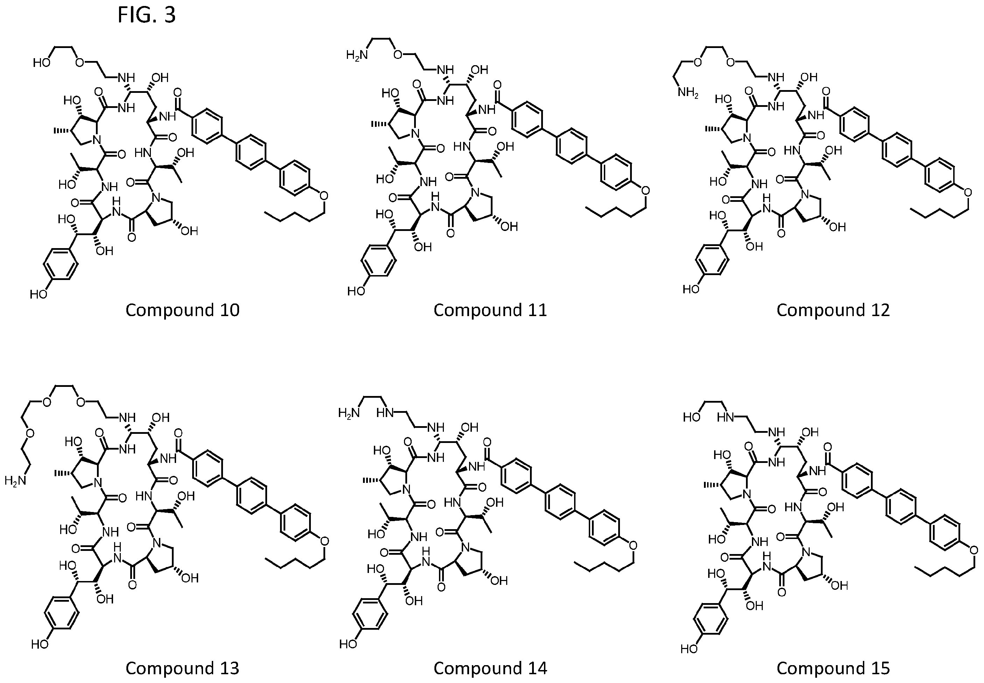

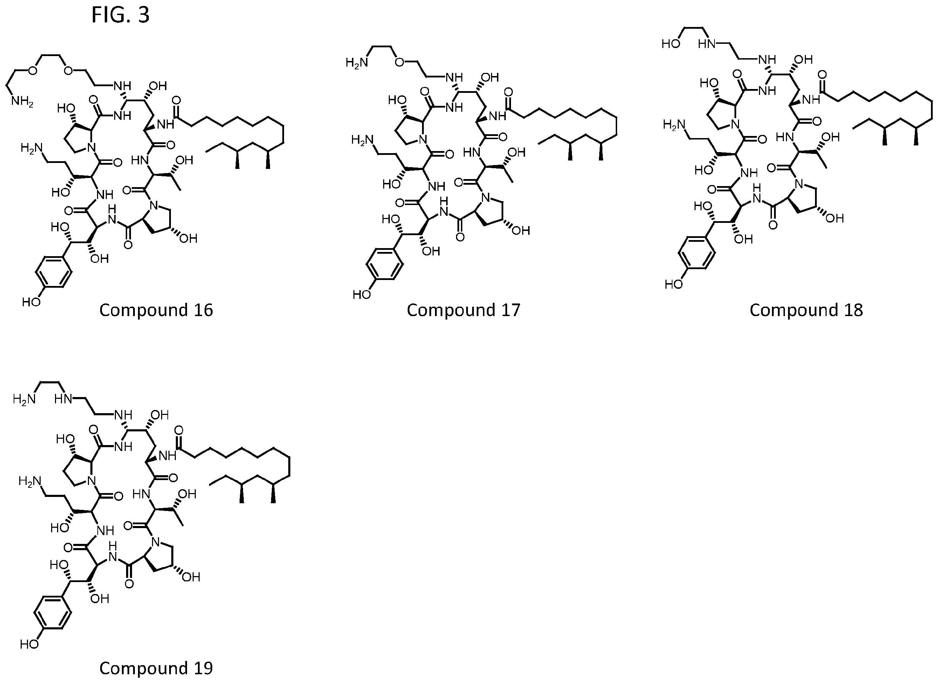

In some embodiments, the compound is selected from Compound 4, Compound 5, Compound 6, Compound 7, Compound 8, Compound 9, Compound 10, Compound 11, Compound 12, Compound 13, Compound 14, Compound 15, Compound 16, Compound 17, Compound 18, Compound 19, and pharmaceutically acceptable salts thereof. The structures of the aforementioned compounds are shown in FIG. 3.

In particular embodiments, the compound is a salt of Compound 2, 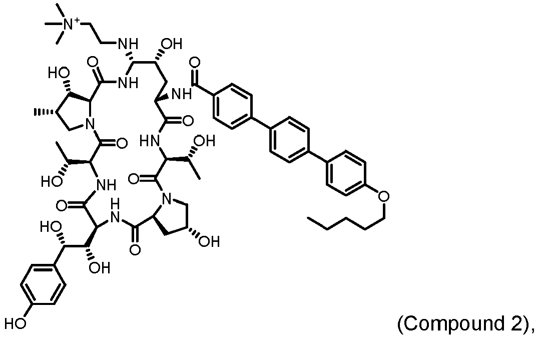 or a neutral form thereof.

In certain embodiments, the compound is Compound 3, 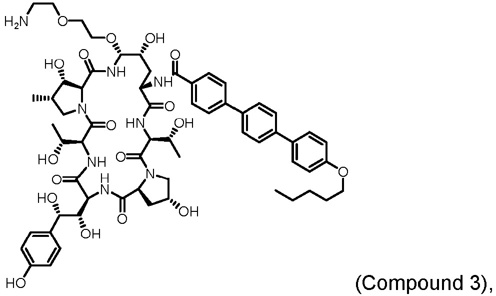 or a pharmaceutically acceptable salt thereof.

The compounds of formulas (I), (II), and (III) can be synthesized, for example, as described in U.S. Patent No. 8,722,619, by coupling functionalized or unfunctionalized echinocandin class compounds with the appropriate acyl, alkyl, hydroxyl, and/or amino groups under standard reaction conditions.

Typically, the semi-synthetic echinocandin class compounds of the disclosure can be made by modifying the naturally occurring echinocandin scaffold. For example, pneumocandin B₀ is prepared by fermentation reactions; where fermentation and mixed broths produce a mixture of products which are then separated to produce pneumocandin B₀, which is used in the synthesis of caspofungin (see U.S. Pat. No. 6,610,822, which describes extraction of the echinocandin class compounds, such as, pneumocandin B₀, WF 11899 and echinocandin B by performing several extraction processes; and see U.S. Pat. No. 6,610,822, which describes methods for purifying the crude extracts).

For semi-synthetic approaches to compounds of the disclosure, the stereochemistry of the compound will be dictated by the starting material. Thus, the stereochemistry of the unnatural echinocandin derivatives will typically have the same stereochemistry as the naturally occurring echinocandin scaffold (representative stereochemistry is depicted in the examples) from which they are derived. Accordingly, any of the compounds shown below anidulafungin, caspofungin, or micafungin can be used as a starting material in the synthesis of the compounds of the disclosure which share the same stereochemical configuration at each of the amino acid residues found in the naturally occurring compound.

Accordingly, the echinocandin class compounds of the disclosure can be derived from the cyclic peptide antifungals which are produced by culturing various microorganisms. For example, amine-terminating compounds can be used to prepare guanidine derivatives. The conversion of amino groups to guanidine groups can be accomplished using standard synthetic protocols. For example, Mosher has described a general method for preparing mono-substituted guanidines by reaction of aminoiminomethanesulfonic acid with amines (Kim, K.; Lin, Y.-T.; Mosher, H. S. Tetrahedron Lett. 29: 3183, 1988). A more convenient method for guanylation of primary and secondary amines was developed by Bernatowicz employing 1H-pyrazole-1-carboxamidine hydrochloride; 1-H-pyrazole-1-(N,N'-bis(tert-butoxycarbonyl)carboxamidine; or 1-H-pyrazole-1-(N,N'-*bis*(benzyloxycarbonyl)carboxamidine. These reagents react with amines to give mono-substituted guanidines (see Bernatowicz et al., J. Org. Chem. 57: 2497, 1992; and Bernatowicz et al., Tetrahedron Lett.34: 3389, 1993). In addition, Thioureas and S-alkyl-isothioureas have been shown to be useful intermediates in the syntheses of substituted guanidines (Poss et al., Tetrahedron Lett. 33: 5933 1992). The compounds that include a heterocyclic ring can be synthesized, for example, by coupling a hydroxyalkyl or aminoalkyl substituted heterocycle with a parent echinocandin compound using those coupling methods described in U.S. Patent No. 8,722,619.

### IV. Pharmaceutical Compositions and Preparations

A compound of any of formulas (I)-(III) (e.g., a salt of Compound 1, or a neutral form thereof) may be prepared in a pharmaceutical composition. In some embodiments, the pharmaceutical composition includes a compound of any of formulas (I)-(III) (e.g., a salt of Compound 1, or a neutral form thereof) and pharmaceutically acceptable carriers and excipients. Depending on the mode of administration and the dosage, a compound of any of formulas (I)-(III) (e.g., a salt of Compound 1, or a neutral form thereof) used in the methods described herein will be formulated into suitable pharmaceutical compositions to permit facile delivery. A compound of any of formulas (I)-(III) (e.g., a salt of Compound 1, or a neutral form thereof) may be formulated in a variety of ways that are known in the art. For use as treatment of human and animal subjects, a compound of any of formulas (I)-(III) (e.g., a salt of Compound 1, or a neutral form thereof) can be formulated as pharmaceutical or veterinary compositions. Depending on the subject to be treated (e.g., a human), the mode of administration, and the type of treatment desired, e.g., prevention, prophylaxis, or therapeutic therapy, a compound of any of formulas (I)-(III) (e.g., a salt of Compound 1, or a neutral form thereof) is formulated in ways consonant with these parameters. A summary of such techniques is found in Remington: The Science and Practice of Pharmacy, 22nd Edition, Lippincott Williams & Wilkins, (2012); and Encyclopedia of Pharmaceutical Technology, eds. J. Swarbrick and J. C. Boylan, 2006, Marcel Dekker, New York.

Acceptable carriers and excipients in the pharmaceutical compositions are nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbenzyl ammonium chloride, resorcinol, and benzalkonium chloride, proteins such as human serum albumin, gelatin, dextran, and immunoglobulins, hydrophilic polymers such as polyvinylpyrrolidone, amino acids such as glycine, glutamine, histidine, and lysine, and carbohydrates such as glucose, mannose, sucrose, and sorbitol.

The pharmaceutical compositions can be prepared in a variety of dosage forms, e.g., intravenous dosage forms, subcutaneous dosage forms, and oral dosage forms (e.g., ingestible solutions, drug release capsules). A compound of any of formulas (I)-(III) (e.g., a salt of Compound 1, or a neutral form thereof) or a pharmaceutical composition of a compound of any of formulas (I)-(III) (e.g., a salt of Compound 1, or a neutral form thereof) may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, preparations suitable for iontophoretic delivery, or aerosols. The compositions may be formulated according to conventional pharmaceutical practice.

The pharmaceutical compositions can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water, physiological saline, and cell culture media (e.g., Dulbecco's Modified Eagle Medium (DMEM), α-Modified Eagles Medium (α-MEM), F-12 medium). Formulation methods are known in the art, see e.g., Gibson (ed.) Pharmaceutical Preformulation and Formulation (2nd ed.) Taylor & Francis Group, CRC Press (2009).

The pharmaceutical compositions can be prepared in the form of an oral formulation. Formulations for oral use include tablets containing the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like. Formulations for oral use may also be provided as chewable tablets, or as hard gelatin capsules wherein the active ingredient is mixed with an inert solid diluent (e.g., potato starch, lactose, microcrystalline cellulose, calcium carbonate, calcium phosphate or kaolin), or as soft gelatin capsules wherein the active ingredient is mixed with water or an oil medium, for example, peanut oil, liquid paraffin, or olive oil. Powders, granulates, and pellets may be prepared using the ingredients mentioned above under tablets and capsules in a conventional manner using, e.g., a mixer, a fluid bed apparatus or a spray drying equipment.

The pharmaceutical composition may be formed in a unit dose form as needed. The amount of active component, e.g., a compound of any of formulas (I)-(III) (e.g., a salt of Compound 1, or a neutral form thereof) included in the pharmaceutical compositions are such that a suitable dose within the designated range is provided (e.g., a dose within the range of 0.01-100 mg/kg of body weight).

### V. Routes, Dosage, and Administration

A compound of any of formulas (I)-(III) or a pharmaceutical composition including a compound of any of formulas (I)-(III) may be formulated for, e.g., intraoral administration, intravenous administration, intramuscular administration, intradermal administration, intraarterial administration, subcutaneous administration, oral administration, or administration by inhalation. In some embodiments, compounds of formulas (I)-(III) or pharmaceutical compositions including a compound of any of formulas (I)-(III) may be formulated for intravenous administration. In some embodiments, Compounds of formulas (I)-(III) or pharmaceutical compositions including a compound of any of formulas (I)-(III) may be formulated for administration by inhalation. In some embodiments, Compounds of formulas (I)-(III) or pharmaceutical compositions including a compound of any of formulas (I)-(III) may be formulated for oral administration. For injectable formulations, various effective pharmaceutical carriers are known in the art. See, e.g., Remington: The Science and Practice of Pharmacy, 22nd ed., (2012) and ASHP Handbook on Injectable Drugs, 18th ed., (2014).

The dosage of a compound of any of formulas (I)-(III) or a pharmaceutical composition including a compound of any of formulas (I)-(III) depends on factors including the route of administration, the disease to be treated, and physical characteristics, e.g., age, weight, general health, of the subject (e.g., a human). The dosage may be adapted by the physician in accordance with conventional factors such as the extent of the disease and different parameters of the subject. Typically, the amount of a compound of any of formulas (I)-(III) or a pharmaceutical composition including a compound of any of formulas (I)-(III) contained within one or more doses may be an amount that effectively reduces the risk of or treats a *Pneumocystis* infection in a subject without inducing significant toxicity.

In any of the embodiments described herein, the compound of formula (I)-(III), or a pharmaceutically acceptable salt thereof, can be administered in doses of about 50 mg to about 1 g (e.g., about 50 mg to about 1 g, about 75 mg to about 950 mg, about 100 mg to about 900 mg, about 125 mg to about 850 mg, about 150 mg to about 800 mg, about 175 mg to about 750 mg, about 200 mg to about 700 mg, about 200 mg to about 400 mg, about 250 mg to about 650 mg, about 300 mg to about 600 mg, about 350 mg to about 550 mg, about 400 mg to about 500 mg, about 425 mg to about 475 mg, e.g., about 150 mg to about 800 mg) of a compound of any of formulas (I)-(III) (e.g., a salt of Compound 1, or a neutral form thereof) are administered to a subject one to three times per week for 2 to 8 weeks (e.g., 2 to 8 weeks, 2 to 7 weeks, 2 to 6 weeks, 2 to 5 weeks, 2 to 4 weeks, or 2 to 3 weeks). In some embodiments, doses of about 50 mg to about 1 g (e.g., about 50 mg to about 1 g, about 75 mg to about 950 mg, about 100 mg to about 900 mg, about 125 mg to about 850 mg, about 150 mg to about 800 mg, about 175 mg to about 750 mg, about 200 mg to about 700 mg, about 250 mg to about 650 mg, about 300 mg to about 600 mg, about 350 mg to about 550 mg, about 400 mg to about 500 mg, about 425 mg to about 475 mg, e.g., about 150 mg to about 800 mg) of a compound of any of formulas (I)-(III) (e.g., a salt of Compound 1, or a neutral form thereof) are administered to a subject one to three times per week for 2 to 8 weeks.

In some embodiments, doses of about 150 mg to about 800 mg of a compound of any of formulas (I)-(III) (e.g., a salt of Compound 1, or a neutral form thereof) are administered to a subject one to three times per week for 2 to 8 weeks (e.g., 2 to 8 weeks, 2 to 7 weeks, 2 to 6 weeks, 2 to 5 weeks, 2 to 4 weeks, or 2 to 3 weeks).

In some embodiments, doses of about 150 mg to about 800 mg of a salt of Compound 1, or a neutral form thereof, are administered to a subject one to three times per week for 2 to 8 weeks.

In some embodiments, a salt of Compound 1, or a neutral form thereof is intravenously administered to a subject (e.g., a human) in two or more weekly doses (e.g., 2, 3, 4, 5, 6, 7, 8, 9, 10, 11, or 12 doses), wherein the first dose contains about 400 mg of a salt of Compound 1, or a neutral form thereof and each of the subsequent doses contains about 200 mg of a salt of Compound 1, or a neutral form thereof. In some embodiments, the first dose includes about 400 mg of Compound 1, or a salt or neutral form thereof, and each of the remaining doses includes about 200 mg of Compound 1, or a salt or neutral form thereof. In some embodiments, the dosing regimen consists of (a) intravenously administering a first dose of about 400 mg of a salt of Compound 1, or a neutral form thereof, (b) intravenously administering a second dose of about 200 mg of a salt of Compound 1, or a neutral form thereof, and (c) optionally intravenously administering a third dose of about 200 mg of a salt of Compound 1, or a neutral form thereof, wherein the first dose is administered on day 1, the second dose is administered on day 8, and the third dose, if administered, is administered on day 15.

In some embodiments, a salt of Compound 1, or a neutral form thereof is intravenously administered to a subject (e.g., a human) in two or more weekly doses (e.g., 2, 3, 4, 5, 6, 7, 8, 9, 10, 11, or 12 doses) of about 400 mg of a salt of Compound 1, or a neutral form thereof. In some embodiments, the dosing regimen consists of (a) intravenously administering a first dose of about 400 mg of a salt of Compound 1, or a neutral form thereof, (b) intravenously administering a second dose of about 400 mg of a salt of Compound 1, or a neutral form thereof, and (c) optionally intravenously administering a third dose of about 400 mg of a salt of Compound 1, or a neutral form thereof, wherein the first dose is administered on day 1, the second dose is administered on day 8, and the third dose, if administered, is administered on day 15.

In any of the embodiments described herein, the timing of the administration of a compound of any of formulas (I)-(III) (e.g., a salt of Compound 1, or a neutral form thereof) depends on the medical and health status of the subject (e.g., a human). In some embodiments, the subject is about to be immunocompromised by a disease, a medical procedure, a drug, and/or a pathogen. In some embodiments, the subject is already immunocompromised by a disease, a medical procedure, a drug, and/or a pathogen. For example, a subject who is about to undergo an immunosuppressive therapy may be administered a compound of any of formulas (I)-(III) (e.g., a salt of Compound 1, or a neutral form thereof) before, during, and/or after receiving the immunosuppresive therapy. The timing of the administration of a compound of any of formulas (I)-(III) (e.g., a salt of Compound 1, or a neutral form thereof) may be optimized by a physician to reduce the risk of or to treat a *Pneumocystis* infection in a subject (e.g., an immunocompromised subject).

The subject (e.g., a human) treated using the methods of the disclosure can be immunocompromised. The immune system of an immunocompromised subject may be weakened or compromised by a disease (e.g., an HIV infection, an autoimmune disease, cancer), a medical procedure (e.g., an organ transplant (e.g., a solid organ transplant) or a bone marrow transplant), a drug (e.g., an immunosuppressant), and/or a pathogen (e.g., bacteria, fungus, virus). In some embodiments, a subject (e.g., an immunocompromised subject) is about to have, is currently having, or has had a disease. In some embodiments, a subject (e.g., an immunocompromised subject) is about to undergo or has undergone a transplantation procedure (e.g., an organ transplant (e.g., a solid organ transplant) or a bone marrow transplant) or a radiation therapy. In some embodiments, a subject (e.g., an immunocompromised subject) is about to be administered, is currently administered, or has been administered one or more drugs that weaken the immune system, e.g., an immunosuppressant (e.g., a corticosteroid). In some embodiments, a subject (e.g., an immunocompromised subject) is about to undergo, is currently undergoing, or has undergone an immunosuppressive therapy, an anti-TNF therapy, a corticosteroid therapy, and/or a chemotherapy. In some embodiments, a subject's immune system is compromised by a pathogen (e.g., bacteria, fungus, virus), i.e., either the pathogen is currently present within the subject or had previously infected the subject. For example, the subject can be HIV positive, have hyper IgM syndrome, or have a CD4⁺ T-cell count of less than 200 cells/µl of blood.

For the treatment of a subject undergoing an immunosuppression therapy, a compound of any of formulas (I)-(III) can be administered concurrently or within a few days (e.g., 1-15 days, e.g., 1, 2, 3, 4, 5, 6, 7, 8, 9, 10, 11, 12, 13, 14, or 15 days (e.g., 7 days)), before and/or after, the subject undergoes an immunosuppression therapy.

For the treatment of a subject undergoing an anti-TNF therapy, a corticosteroid therapy, or a chemotherapy a compound of any of formulas (I)-(III) can be administered concurrently or within a few days (e.g., 1-15 days, e.g., 1, 2, 3, 4, 5, 6, 7, 8, 9, 10, 11, 12, 13, 14, or 15 days (e.g., 7 days)), before and/or after, the subject undergoes an anti-TNF therapy, a corticosteroid therapy, or a chemotherapy.

For the treatment of a subject undergoing a transplantation procedure (e.g., an organ transplant (e.g., a solid organ transplant) or a bone marrow transplant), a compound of any of formulas (I)-(III) can be administered concurrently or within a few days (e.g., 1-15 days, e.g., 1, 2, 3, 4, 5, 6, 7, 8, 9, 10, 11, 12, 13, 14, or 15 days (e.g., 7 days)), before and/or after, the subject undergoes a transplantation procedure (e.g., an organ transplant (e.g., a solid organ transplant) or a bone marrow transplant). In some embodiments, the treatment may start before and continue after the organ transplant.

In any of the embodiments described herein, a second antifungal agent can be used in combination with a compound of any of formulas (I)-(III) (e.g., a salt form of Compound 1) to prevent or treat a *Pneumocystis* infection (e.g., a PCP infection). The second antifungal agent and the compound of any of formulas (I)-(III) can be administered concurrently. Alternatively, the compound of any of formulas (I)-(III) can be administered first, followed by administration of the second antifungal agent. In some embodiments, the second antifungal agent is administered first, followed by administering of the compound of any of formulas (I)-(III).

Examples of second antifungal agents that can be used in the combination therapies described herein include, but are not limited to, clindamycin (sold under the brand names CLEOCIN^{®} and DALACIN^{®}), trimethoprim (sold under the brand names PROLOPRIM^{®}, MONOTRIM^{®}, and TRIPRIM^{®}), sulfamethoxazole (sold under the brand name GANTANOL^{®}), cotrimoxazole (a combination of trimethoprim and sulfamethoxazole (aka TMP-SMX); this combination is sold under the brand names BACTRIM^{®}, COTRIM^{®}, SULFATRIM ^{®}, and SEPTRA^{®}), atovaquone (sold under the brand name MEPRON^{®}), pentamidine (sold under the brand names NEBUPENT^{®} and PENTRAM^{®}), primaquine, pyrimethamine (sold under the brand name DARAPRIM^{®}), and pharmaceutically acceptable salts thereof.

Alternatively, the second antifungal agents that can be used in the combination therapies described herein is selected from glucan synthase inhibitors (e.g., echinocandins, enfumafungins), polyene compounds, azole compounds, and pharmaceutically acceptable salts thereof.

Examples of glucan synthase inhibitors that can be used in the combination therapies of the disclosure include, but are not limited to echinocandins (e.g., caspofungin, micafungin, or anidulafungin) enfumafungin (e.g., SCY-078 (aka MK-3118, see Lepak et al., Antimicrobial agents and chemotherapy 59:1265 (2015)), and pharmaceutically acceptable salts thereof.

The azole compounds are antifungal compounds that contain an azole group (i.e., a five-membered heterocyclic ring having at least one N and one or more heteroatoms selected from N, O, or S). Azole compounds function by binding to the enzyme 14α-demethylase and disrupt, inhibit, and/or prevent its natural function. The enzyme 14α-demethylase is a cytochrome P450 enzyme that catalyzes the removal of the C-14 α-methyl group from lanosterol before lanosterol is converted to ergosterol, an essential component in the fungal cell wall. Therefore, by inhibiting 14α-demethylase, the synthesis of ergosterol is inhibited. Examples of azole compounds that can be used in the combination therapies of the disclosure include, but are not limited to (e.g., VT-1161, VT-1129, VT-1598, fluconazole, albaconazole, bifonazole, butoconazole, clotrimazole, econazole, efinaconazole, fenticonazole, isavuconazole, isoconazole, itraconazole, ketoconazole, luliconazole, miconazole, omoconazole, oxiconazole, posaconazole, pramiconazole, ravuconazole, sertaconazole, sulconazole, terconazole, tioconazole, and voriconazole), VL-2397, and flucytosine (ANCOBON^{®}).

Polyene compounds are compounds that insert into fungal membranes, bind to ergosterol and structurally related sterols in the fungal membrane, and disrupt membrane structure integrity, thus causing leakage of cellular components from a fungus that causes infection. Polyene compounds typically include large lactone rings with three to eight conjugated carbon-carbon double bonds and may also contain a sugar moiety and an aromatic moiety. Polyene compounds typically include large lactone rings with three to eight conjugated carbon-carbon double bonds and may also contain a sugar moiety and an aromatic moiety. Examples of polyene compounds that can be used in the combination therapies of the disclosure include, but are not limited to, 67-121-A, 67-121-C, amphotericin B, derivatives of amphotericin B (e.g., C35deOAmB; see Gray et al., Proceedings of the National Academy of Sciences 109:2234 (2012)), arenomvcin B, aurenin, aureofungin A, aureotuscin, candidin, chinin, chitin synthesis inhibitors (e.g., lufenuron), demethoxyrapamycin, dermostatin A, dermostatin B, DJ-400-B₁, DJ-400-B₂, elizabethin, eurocidin A, eurocidin B, filipin I, filipin II, filipin III, filipin IV, fungichromin, gannibamycin, hamycin, levorin A₂, lienomycin, lucensomycin, mycoheptin, mycoticin A, mycoticin B, natamycin, nystatin A, nystatin A₃, partricin A, partricin B, perimycin A, pimaricin, polifungin B, rapamycin, rectilavendomvcin, rimocidin, roflamycoin, tetramycin A, tetramycin B, tetrin A, tetrin B, and pharmaceutically acceptable salts thereof.

The following Examples are intended to further illustrate but not to limit the disclosure herein.

### EXAMPLES

### Example 1 - Efficacy of Compound 1 to prevent Pneumocystis murina infection in vivo

The *in vivo* efficacy of Compound 1 in preventing *Pneumocystis murina* infection in immunocompromised mice was evaluated.

### Compound 1 preparation

Compound 1 (as its acetate salt) was added to a vehicle solution. Briefly, the vehicle was prepared by first adding 1% Tween 20 to sterile phosphate-buffered saline (PBS) and then adding DMSO for a 10% DMSO in 1% Tween 20 in sterile PBS solution. Vehicle was stored in the refrigerator and aliquots were allowed to warm to room temperature before mixing with Compound 1. Drug preparations were prepared by adding Compound 1 to vehicle based on drug dosages and an average mouse weight of 24g. For the 20 mg/kg/d dose, the drug was dissolved in vehicle to a concentration of 2.4 mg/ml. The mice then received 0.48 mg in a 200 µl dose. The drug was stored at -20 °C until initiation of dosage. The drug was prepared fresh for every two days of dosing.

**Table 1. Pneumocystis murina (P. murina) prophylaxis study design.**

| **Group** | **Drug** | **Dose** | **# of Animals** |
|---|---|---|---|
| 1 | Negative Control | --- | 10 |
| 2 | Compound 1 | 20 mg/kg/3x/wk | 10 |
| 3 | Compound 1 | 20 mg/kg/1x/wk | 10 |
| 4 | Compound 1 | 2 mg/kg/3x/wk | 10 |
| 5 | Compound 1 | 2m4/kg/1x/wk | 10 |
| 6 | Compound 1 | 0.2 mg/kg/3x/wk | 10 |
| 7 | Compound 1 | 0.2 mg/kg/1x/wk | 10 |
| 8 | Compound 1 | 50/250 mg/kg/3x/wk | 10 |

### Experimental method

C3H/HeN mice (Charles River) were infected with *P. murina* by intranasal inoculation of *P*. *murina* organisms at 2 x 106/50 µl from our liquid nitrogen repository. Prior to inoculation, the *P*. *murina* organisms were pre-incubated overnight in RPMI 1640 medium supplemented with calf serum and antibiotics to eliminate any bacterial contamination. The immune systems of the mice were suppressed by the addition of dexamethasone at 4 mg/liter to acidified drinking water (sulfuric acid at 1 ml/liter). Acidification is used to prevent secondary microbial infections. The mice were divided into a negative control group (control steroid - C/S) (Group 1 in Table 1), positive control group (trimethoprim/sulfamethoxazole - TMP/SMX) (Group 8 in Table 1), and treatment groups (Groups 2-7 in Table 1). Drugs to be tested were administered intraperitoneally (i.p.) on a mg/kg/d basis; dose, route, and frequency of administration will vary depending on the agent being tested. For the prophylaxis study, Compound 1 was administered at the same time the mice were inoculated. Immune suppression and treatment continued for the entire 6-week study. No complications were noted during the course of this study. The mice tolerated the dosing regimen with no significant deaths observed. At the end of the study, the mice were euthanized by CO₂ and their lungs processed for analysis by homogenization. Slides were made from the lung homogenates at different dilutions and stained with Diff-Quik to quantify the trophic forms and cresyl echt violet to quantify the asci.

### Calculations

Efficacy is based on the reduction of organism burden between the treatment groups and the negative control group as determined by microscopic evaluation. The nuclei and asci counts for each lung are log transformed and statistical analysis is determined by the analysis of variance (ANOVA); individual groups are compared by the Student-Newman-Keuls t test for multiple comparisons using GraphPad Prism. Statistical significance is accepted at a p value <0.05.

### Results

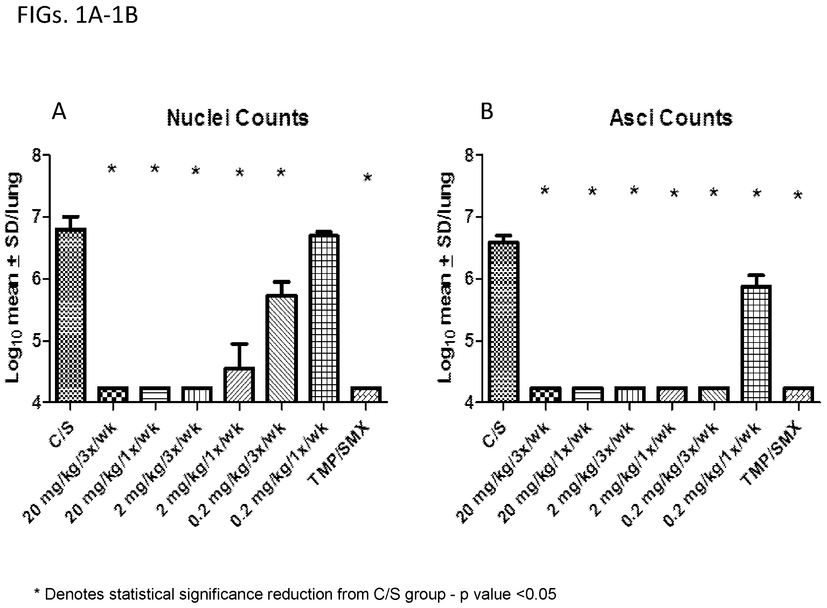

Prophylactic treatment of *P*. *murina* infected mice with Compound 1 showed a statistically significant reduction in nuclei levels at all dose levels except for the 0.2 mg/kg/1x/week group as compared to the negative control group after 6 weeks of dosing (FIG. 1A). Three of the treatment groups (20 mg/kg/1x/week, 20 mg/kg/1x/week, and 2 mg/kg/3x/week groups) were equally as efficacious as the positive control TMP/SMX group with no nuclei seen by microscopic evaluation. All Compound 1 treatment groups showed a statistically significant reduction in asci levels compared to the negative control group (FIG. 1B) and there was no difference in efficacy between 5 treatment groups and the positive control TMP/SMX group with no asci observed by microscopic evaluation.

### Example 2 - Efficacy of Compound 1 to treat Pneumocystis murina infection in vivo

The *in vivo* efficacy of Compound 1 in treating *Pneumocystis murina* infection in immunocompromised mice was evaluated.

### Compound 1 preparation

Compound 1 was added to a vehicle solution. Briefly, the vehicle was prepared by first adding 1% Tween 20 to sterile phosphate-buffered saline (PBS) and then adding DMSO for a 10% DMSO in 1% Tween 20 in sterile PBS solution. Vehicle was stored in the refrigerator and aliquots were allowed to warm to room temperature before mixing with Compound 1. Drug preparations were prepared by adding Compound 1 to vehicle based on drug dosages and an average mouse weight of 24 g. For the 20 mg/kg/d dose, the drug was dissolved in vehicle to a concentration of 2.4 mg/ml. The mice then received 0.48 mg in a 200 µl dose. The drug was stored at -20 °C until initiation of dosage. The drug was prepared fresh for every two days of dosing.

**Table 2. P. murina treatment study design.**

| **Group** | **Drug** | **Dose** | **Treatment Days** | **# of Animals** |
|---|---|---|---|---|
| 1 | Negative Control | --- | 7 | 10 |
| 2 | Compound 1 | 20 mg/kg/d | 7 | 10 |
| 3 | Compound 1 | 2 mg/kg/d | 7 | 10 |
| 4 | Compound 1 | 0.2 mg/kg/d | 7 | 10 |
| 5 | TMP/SMX | 50/250 mg/kg/d | 7 | 8 |

### Experimental method

C3H/HeN mice (Charles River) were infected with *P. murina* pneumonia by intranasal inoculation of *P*. *murina* organisms from a liquid nitrogen repository at 2 x 10⁶/50 µl. Prior to inoculation, the *P*. *murina* organisms were pre-incubated overnight in RPMI 1640 medium supplemented with calf serum and antibiotics to eliminate any bacterial contamination. The immune systems of the mice were suppressed by treatment with the anti-CD4⁺ antibody GK 1.5. 300 µg of GK 1.5 was administered intraperitoneally 3x in week 1 prior to inoculating the mice. After inoculation, the GK 1.5 was administered 1x/week for remainder of the study. Mice were fed sterilized lab chow and supplied with acidified water (sulfuric acid at 1 ml/liter) to prevent secondary microbial infections.

After the mice developed a moderate infection level (approximately 5 weeks), they were divided into a negative control group (control steroid - C/S) (Group 1 in Table 2), positive control group (trimethoprim/sulfamethoxazole - TMP/SMX) (Group 5), and treatment groups (Groups 2-4). Drugs to be tested were administered intraperitoneally (i.p.) on a mg/kg/d basis for one week; dose, route, and frequency of administration varied depending on the agent being tested. The mice were euthanized by CO₂ at day 7. The mice were sacrificed and their lungs processed for analysis by homogenization. Slides were made from the lung homogenates at different dilutions and stained with Diff-Quik to quantify the trophic forms and cresyl echt violet to quantify the asci.

### Calculations

Efficacy is based on the reduction of organism burden between the treatment groups and the negative control group as determined by microscopic evaluation. The nuclei and asci counts for each lung are log transformed and statistical analysis is determined by the analysis of variance (ANOVA); individual groups are compared by the Student-Newman-Keuls t test for multiple comparisons using GraphPad Prism v6. Statistical significance is accepted at a p value <0.05.

### Results

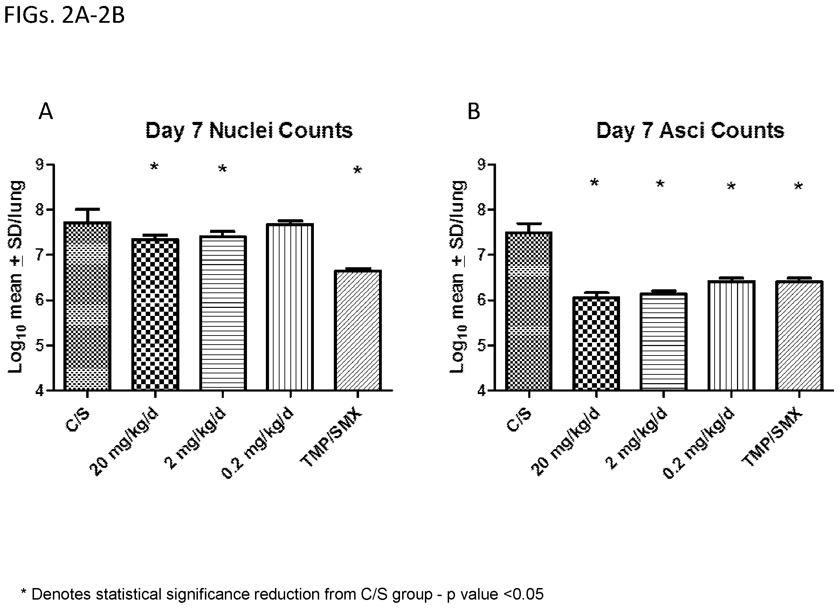

Treatment of *P*. *murina* infected mice with Compound 1 showed a statistically significant reduction in nuclei levels in the 20 mg/kg/d and 2 mg/kg/d treatment groups compared to the negative control group after 7 days of dosing (FIG. 2A). All Compound 1 treatment groups showed a statistically significant reduction in asci levels compared to the negative control group after 7 days (FIG. 2B) and there was no difference in efficacy between these 3 treatment groups and the positive control TMP/SMX group.

### Example 3 - Treatment of a Pneumocystis pneumonia (PCP) in an immunocompromised subject.

An immunocompromised HIV positive human subject presenting with fever, cough, shortness of breath, weight loss, and/or night sweats is diagnosed with PCP using any one of a variety of methods (e.g., chest x-ray, gallium 67 scans). The diagnosis is confirmed by histological identification of the causative organism in sputum or bronchio-alveolar lavage.

The subject commences treatment with 200 - 600 mg (e.g., 400 mg) of the acetate salt of Compound 1 intravenously administered once weekly for 2 - 12 weeks.

The CD4⁺ T-cell count of the subject is monitored as the subject undergoes anti-HIV treatment to strengthen the immune function of the subject.

Weekly treatment with compound 1 is continued until the CD4⁺ T-cell count of the subject reaches a predetermined level (e.g., greater than 600 cells/µl of blood).

### Example 4 - Prophylaxis against Pneumocystis pneumonia infection in a subject.

A human subject is scheduled to undergo a kidney transplant. As part of that procedure, the subject receives an immunosuppressant to prevent the emergence of Graft-versus-host disease.

Seven days prior to commencing immunosuppression therapy, the subject commences treatment with 200 - 600 mg (e.g., 400 mg) of the acetate salt of Compound 1 intravenously administered once weekly for 2 - 12 weeks.

Weekly treatment with compound 1 is continued until the subject is weaned from the immunosuppressant, or until normal immune function is restored.

### Example 5 - Pharmacological Basis of Compound 1 Efficacy

### Methods

*Pharmacokinetic Study.* Healthy female ICR mice were given a single dose of Compound 1 via intraperitoneal (IP) injection. The following doses, at three mice per dose, were studied: 1, 4, and 16 mg/kg. Compound 1 plasma concentrations were determined at 0, 1, 3, 6, 12, 24, 48, 72, 96 hours post-dose using a validated LC/MS assay with a lower limit of quantification of 0.02 µg/mL.

*Dose-Fractionation Study.* Male or female ICR mice (5 per regimen and observation time) weighing 22 ± 2 g were rendered neutropenic for the study by injecting the mice with cyclophosphamide treatment four days (- Day 4) (150 mg/kg IP) and one day (- Day 1) prior to infection at 100 mg/kg IP. Neutropenia was sustained for the duration of the study with cyclophosphamide doses (100 mg/kg IP) every 48 hours on days +1, +3, + 5 and + 7 after infection. Each animal was inoculated intravenously with 1 x 10³ CFU of *Candida albicans* (Strain R303, MIC=0.125 mg/L). Compound 1 (or vehicle) was administered 24 hours post-infection via IP injection. The doses studied are shown in Table 3.

**Table 3. Summary of Compound 1 dosing regimens evaluated**

| **Total Dose** | **Dosing Interval** | **Fractionated Doses** |
|---|---|---|
| 0.7 mg/kg | Single Dose | 0.7 mg/kg x 1 |
| | Twice Weekly | 0.35 mg/kg x 2 |
| | Daily | 0.1 mg/kg x 7 |
| 2 mg/kg | Single Dose | 2 mg/kg x 1 |
| | Twice Weekly | 1 mg/kg x 2 |
| | Daily | 0.29 mg/kg x 7 |
| 7 mg/kg | Single Dose | 7 mg/kg x 1 |
| | Twice Weekly | 3.5 mg/kg x 2 |
| | Daily | 1 mg/kg x 7 |

Mice were sacrificed 168 hours (7 days) following the start of treatment. Control arm mice were sacrificed 0, 24, and 48 hours post administration of vehicle. Paired kidneys are aseptically harvested, homogenized, and plated for colony counts to determine the fungal burden (CFU/g).

*Pharmacokinetic-Pharmacodynamic Analyses.* Using the data collected from the PK study, a PK model was developed in S-ADAPT. Using the developed PK model, concentration-time profiles and AUC₀₋₁₆₈ₕ values were computed for each dosing regimen administered in the dose-fractionation study. Free-drug plasma concentrations were generated using a murine protein binding value of 99.1%. Relationships between the change in log₁₀ CFU from start of therapy and AUC₀₋₁₆₈ₕ were explored.

### Results

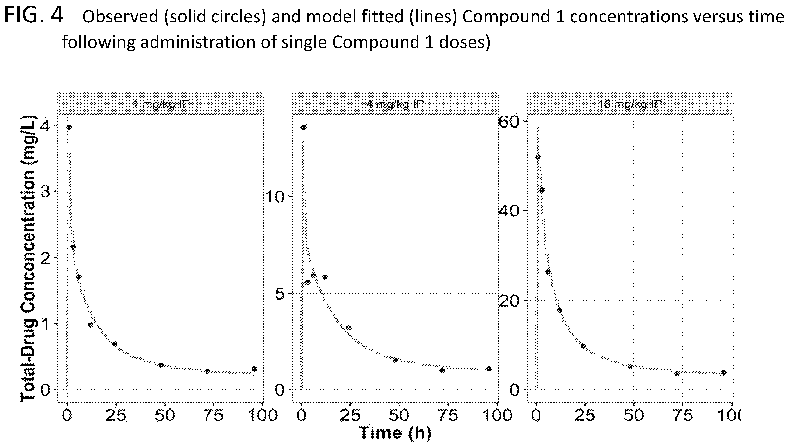

Compound 1 exhibited linear PK over the dose ranged studied (1 to 16 mg/kg IP). A 4-compartment model best described the PK data. Model fits are displayed in FIG. 4.

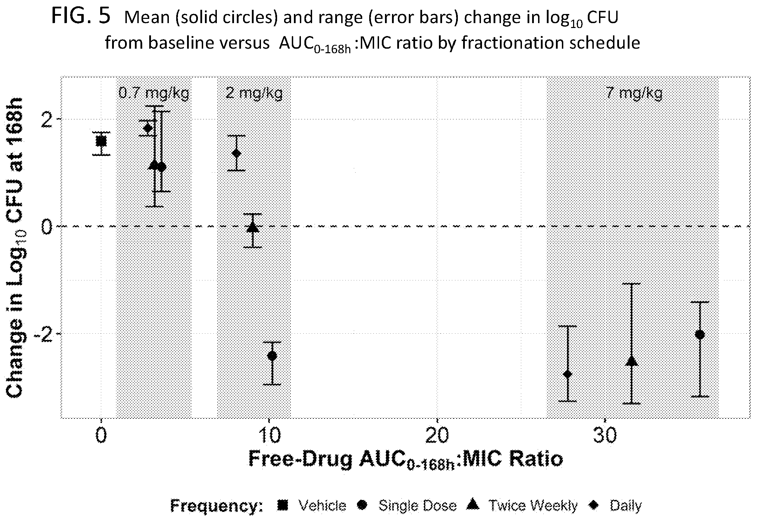

The results of the dose-fractionation study are displayed in FIG. 5, which shows that fungi grew well in the no-treatment control group. The magnitude of net change in fungal density (log₁₀ CFU) was similar regardless of fractionation schedule within the Compound 1 0.7 and 7 mg/kg dosing groups. However, results within the Compound 1 2 mg/kg group varied by the fractionation schedule.

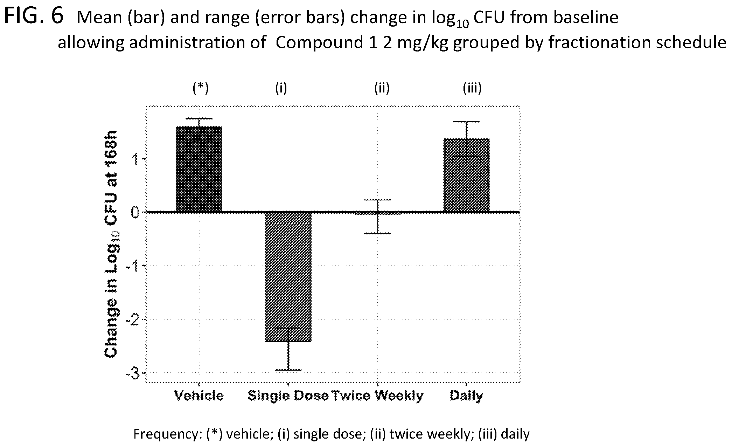

The change in log₁₀ CFU reduction from baseline at 168 hours by fractionation schedule for the Compound 1 2 mg/kg group is displayed in FIG. 6. When a total dose of 2 mg/kg was delivered daily (0.29 mg/kg/day), the magnitude of net change in fungal density (log₁₀ CFU) was similar to the no-treatment control group. However, when 2 mg/kg is delivered as a single dose, there was a greater than 2-log₁₀ CFU reduction from baseline at 168 hours. The 2 mg/kg x1 and 0.29 mg/kg daily x 7 regimens had similar cumulative Compound 1 exposures at 168 hours, as displayed in FIG. 5. Despite having similar exposures, which influences efficacy, these regimens showed very different effects.

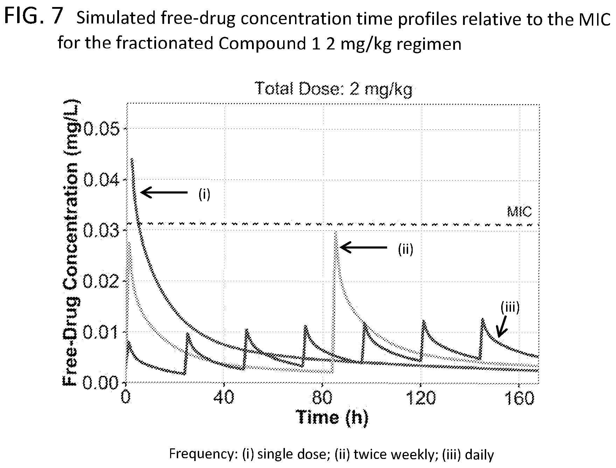

Free-drug plasma concentration-time profiles of the three fractionated Compound 1 2 mg/kg dosing regimens are displayed in FIG. 7. All three regimens display very different exposure profiles. In particular, the single dose regimen results in larger Compound 1 exposures early in therapy. Free-drug plasma AUC₀₋₂₄ is 0.0654, 0.0303, and 0.00948 mg·h/L following administration of Compound 1 2 mg/kg as a single dose, twice weekly, and daily regimen, respectively. Further, administration of a single dose results in free-drug plasma concentrations which remain above those for the twice weekly and daily regimens for 84 and 48 hours, respectively.

Three Compound 1 regimens with similar total exposures, yet very different exposure shapes, display considerably different efficacy. This suggests that the shape of the Compound 1 AUC is a determinant of efficacy, with front loaded regimens demonstrating greater efficacy. The magnitude of the net change in fungal burden was similar regardless of fractionation schedule within the Compound 1 0.7 and 7 mg/kg dosing groups, but differed within the 2 mg/kg group. A 2 mg/kg dose was considerably more effective when given once per week compared to the same dose divided into twice-weekly or daily regimens.

## Claims

1. A compound for use in a method of treating or reducing the risk of a *Pneumocystis* infection in a subject, the method comprising administering to the subject the compound in an amount and for a duration sufficient to treat the *Pneumocystis* infection, wherein the compound is Compound 1, or a pharmaceutically acceptable salt thereof.

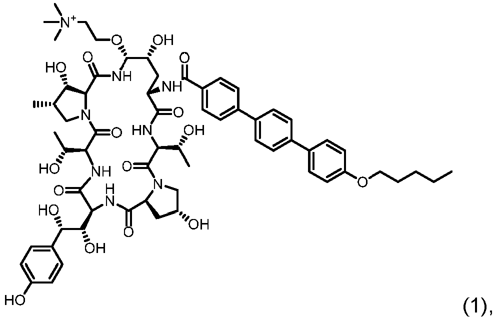

2. The compound for use according to claim 1, wherein the method of treating comprises reducing the cyst burden in the lung of the subject or inhibiting the reproduction of a *Pneumocystis* spp. in the subject.

3. The compound for use according to claims 1 or 2, wherein the subject has *Pneumocystis* pneumonia.

4. The compound for use according to any one of claims 1-3, wherein:
a) the subject is immunocompromised; or:
b) the subject is HIV positive or has hyper IgM syndrome; and/or:
c) the subject has a CD4⁺ T-cell count of less than 200 cells/µl of blood.

5. The compound for use according to any one of claims 1-4, wherein Compound 1, or a pharmaceutically acceptable salt thereof, is administered concurrently or within 7 days, before and/or after, the subject:
a) undergoes an immunosuppression therapy; or:
b) undergoes an anti-TNF therapy, a corticosteroid therapy, or a chemotherapy; or:
c) undergoes a transplantation procedure; optionally wherein the transplantation procedure is an organ transplant or a bone marrow transplant.

6. The compound for use according to any one of claims 1-5, wherein the subject is a human and the *Pneumocystis* infection is caused by *Pneumocystis jirovecii.*

7. The compound for use according to any one of claims 1-6, wherein the compound is administered intraorally, intravenously, intramuscularly, intradermally, intraarterially, subcutaneously, orally, or by inhalation.

8. A compound for use in an *in vivo* method of inhibiting the replication of a *Pneumocystis* spp. comprising contacting the *Pneumocystis* spp. with said compound in an amount and for a duration sufficient to inhibit replication of the *Pneumocystis* spp., wherein the compound is Compound 1: 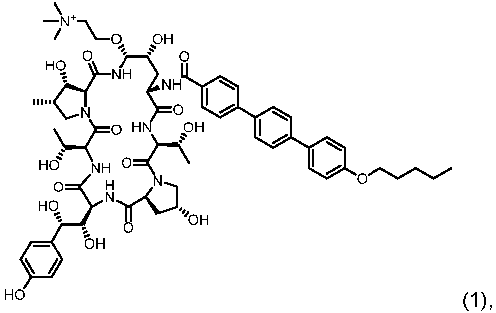 or a pharmaceutically acceptable salt thereof.

9. The compound for use according to claim 8, wherein the *Pneumocystis* spp. is *Pneumocystis jirovecii.*

10. The compound for use according to claim 1-9 wherein the method comprises administering to the subject doses of about 150 mg to about 800 mg of a salt of Compound 1, or a neutral form thereof, one to three times per week to the subject for 2 to 8 weeks.

11. The compound for use according to any one of claims 1-10, wherein the compound is administered concurrently with a second antifungal agent; optionally wherein:
a) the second antifungal agent is clindamycin, trimethoprim, sulfamethoxazole, cotrimoxazole, atovaquone, pentamidine, primaquine, pyrimethamine, or a pharmaceutically acceptable salt thereof; or:
b) the second antifungal agent is selected from glucan synthase inhibitors, polyene compounds, azole compounds, and pharmaceutically acceptable salts thereof.

12. The compound for use according to any one of claims 1-11, wherein the method comprises once weekly dosing of Compound 1 in salt or neutral form, the method comprising
(a) intravenously administering a first dose of about 400 mg of Compound 1 in salt or neutral form,
(b) intravenously administering a subsequent dose of about 200 mg or about 400 mg of Compound 1 in salt or neutral form, and
(c) optionally repeating step (b) for one to ten weeks.

13. The compound for use according to claim 12, wherein the method consists of
(a) intravenously administering a first dose of about 400 mg of Compound 1 in salt or neutral form,
(b) intravenously administering a second dose of 200 mg of Compound 1 in salt or neutral form, and
(c) optionally intravenously administering a third dose of about 200 mg of Compound 1 in salt or neutral form,
wherein the first dose is administered on day 1, the second dose is administered on day 8, and the third dose, if administered, is administered on day 15;
or wherein the method consists of
(a) intravenously administering a first dose of about 400 mg of Compound 1 in salt or neutral form,
(b) intravenously administering a second dose of about 400 mg of Compound 1 in salt or neutral form, and
(c) optionally intravenously administering a third dose of about 400 mg of Compound 1 in salt or neutral form,
wherein the first dose is administered on day 1, the second dose is administered on day 8, and the third dose, if administered, is administered on day 15.

14. The compound for use according to any one of claims 1-13, wherein:
a) Compound 1 is administered over a time period of 30 to 180 minutes; and/or;
b) Compound 1 is administered as an aqueous pharmaceutical composition; optionally wherein the pharmaceutical composition has a pH of from 4.0 to 8.

15. The compound for use according to any one of claims 1-14, wherein the Compound 1 salt is Compound 1 acetate.

## Patentansprüche

1. Verbindung zur Verwendung bei einem Verfahren zur Behandlung oder Verringerung des Risikos einer Pneumocystis-Infektion bei einem Individuum, wobei das Verfahren Verabreichen der Verbindung in einer Menge und hinreichend lange zur Behandlung der *Pneumocystis-*Infektion an das Individuum umfasst, wobei es sich bei der Verbindung um Verbindung 1 oder ein pharmazeutisch unbedenkliches Salz davon handelt.

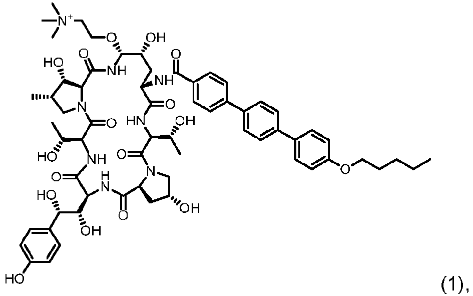

2. Verbindung zur Verwendung nach Anspruch 1, wobei das Behandlungsverfahren das Verringern der Zystenlast in der Lunge des Individuums oder das Hemmen der Vermehrung einer *Pneumocystis* spp. bei dem Individuum umfasst.

3. Verbindung zur Verwendung nach Anspruch 1 oder 2, wobei das Individuum an Pneumocystis-Lungenentzündung leidet.

4. Verbindung zur Verwendung nach einem der Ansprüche 1-3, wobei:
a) das Individuum immungeschwächt ist; oder:
b) das Individuum HIV-positiv ist oder an Hyper-IgM-Syndrom leidet; und/oder:
c) das Individuum eine CD4⁺-T-Zellzahl von weniger als 200 Zellen/µl Blut aufweist.

5. Verbindung zur Verwendung nach einem der Ansprüche 1-4, wobei Verbindung 1 oder ein pharmazeutisch unbedenkliches Salz davon gleichzeitig oder innerhalb von 7 Tagen verabreicht wird, bevor und/oder nachdem das Individuum:
a) einer Immunsuppressionstherapie unterzogen wird; oder:
b) einer Anti-TNF-Therapie, einer Corticosteroidtherapie oder einer Chemotherapie unterzogen wird; oder:
c) einem Transplantationseingriff unterzogen wird; gegebenenfalls wobei es sich bei dem Transplantationseingriff um eine Organtransplantation oder eine Knochenmarktransplantation handelt.

6. Verbindung zur Verwendung nach einem der Ansprüche 1-5, wobei es sich bei dem Individuum um einen Menschen handelt und die *Pneumocystis*-Infektion durch *Pneumocystis jirovecii* verursacht wird.

7. Verbindung zur Verwendung nach einem der Ansprüche 1-6, wobei die Verbindung intraoral, intravenös, intramuskulär, intradermal, intraarteriell, subkutan, oral oder per Inhalation verabreicht wird.

8. Verbindung zur Verwendung bei einem In-vivo-Verfahren zur Hemmung der Replikation einer *Pneumocystis* spp., umfassend Inkontaktbringen der *Pneumocystis* spp. mit der Verbindung in einer Menge und hinreichend lange zur Hemmung der Replikation der *Pneumocystis* spp., wobei es sich bei der Verbindung um Verbindung 1: 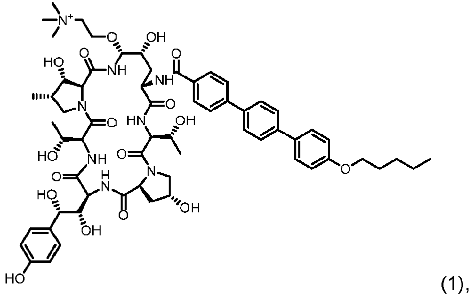 oder ein pharmazeutisch unbedenkliches Salz davon handelt.

9. Verbindung zur Verwendung nach Anspruch 8, wobei es sich bei der *Pneumocystis* spp. um *Pneumocystis jirovecii* handelt.

10. Verbindung zur Verwendung nach Anspruch 1-9, wobei das Verfahren Verabreichen an das Individuum von Dosen von etwa 150 mg bis etwa 800 mg eines Salzes von Verbindung 1 oder einer neutralen Form davon ein- bis dreimal pro Woche an das Individuum über 2 bis 8 Wochen umfasst.

11. Verbindung zur Verwendung nach einem der Ansprüche 1-10, wobei die Verbindung gleichzeitig mit einem zweiten Antimykotikum verabreicht wird; gegebenenfalls wobei:
a) es sich bei dem zweiten Antimykotikum um Clindamycin, Trimethoprim, Sulfamethoxazol, Cotrimoxazol, Atovaquon, Pentamidin, Primaquin, Pyrimethamin oder ein pharmazeutisch unbedenkliches Salz davon handelt; oder:
b) das zweite Antimykotikum aus Glucansynthase-Inhibitoren, Polyen-Verbindungen, Azolverbindungen und pharmazeutisch unbedenklichen Salzen davon ausgewählt ist.

12. Verbindung zur Verwendung nach einem der Ansprüche 1-11, wobei das Verfahren Dosieren von Verbindung 1 einmal wöchentlich in Salz- oder neutraler Form umfasst, wobei das Verfahren Folgendes umfasst:
(a) intravenöses Verabreichen einer ersten Dosis von etwa 400 mg Verbindung 1 in Salz- oder neutraler Form,
(b) intravenöses Verabreichen einer Folgedosis von etwa 200 mg oder etwa 400 mg Verbindung 1 in Salz- oder neutraler Form und
(c) gegebenenfalls Wiederholen von Schritt (b) über eine bis zehn Wochen.

13. Verbindung zur Verwendung nach Anspruch 12, wobei das Verfahren aus Folgendem besteht:
(a) intravenöses Verabreichen einer ersten Dosis von etwa 400 mg Verbindung 1 in Salz- oder neutraler Form,
(b) intravenöses Verabreichen einer zweiten Dosis von 200 mg Verbindung 1 in Salz- oder neutraler Form und
(c) gegebenenfalls intravenöses Verabreichen einer dritten Dosis von etwa 200 mg Verbindung 1 in Salz- oder neutraler Form,
wobei die erste Dosis an Tag 1, die zweite Dosis an Tag 8 und die dritte Dosis, falls verabreicht, an Tag 15 verabreicht wird;
oder wobei das Verfahren aus Folgendem besteht:
(a) intravenöses Verabreichen einer ersten Dosis von etwa 400 mg Verbindung 1 in Salz- oder neutraler Form,
(b) intravenöses Verabreichen einer zweiten Dosis von etwa 400 mg Verbindung 1 in Salz- oder neutraler Form und
(c) gegebenenfalls intravenöses Verabreichen einer dritten Dosis von etwa 400 mg Verbindung 1 in Salz- oder neutraler Form,
wobei die erste Dosis an Tag 1, die zweite Dosis an Tag 8 und die dritte Dosis, falls verabreicht, an Tag 15 verabreicht wird.

14. Verbindung zur Verwendung nach einem der Ansprüche 1-13, wobei:
a) Verbindung 1 über einen Zeitraum von 30 bis 180 Minuten verabreicht wird; und/oder;
b) Verbindung 1 als wässrige pharmazeutische Zusammensetzung verabreicht wird; gegebenenfalls wobei die pharmazeutische Zusammensetzung einen pH-Wert von 4,0 bis 8 aufweist.

15. Verbindung zur Verwendung nach einem der Ansprüche 1-14, wobei es sich bei dem Verbindung-1-Salz um Verbindung-1-Acetat handelt.

## Revendications

1. Composé pour utilisation dans un procédé de traitement ou de réduction du risque d'une infection par Pneumocystis chez un sujet, le procédé comprenant l'administration au sujet du composé en une quantité et pendant une durée suffisantes pour traiter l'infection par Pneumocystis, le composé étant le composé 1, ou un sel pharmaceutiquement acceptable de celui-ci.

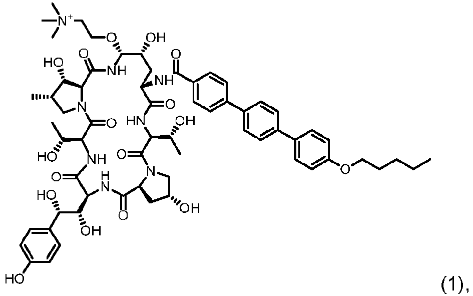

2. Composé pour utilisation selon la revendication 1, dans lequel le procédé de traitement comprend la réduction de la charge kystique dans le poumon du sujet ou l'inhibition de la reproduction d'un Pneumocystis spp. chez le sujet.

3. Composé pour utilisation selon les revendications 1 ou 2, dans lequel l'anticorps présente une pneumonie à Pneumocystis.

4. Composé pour utilisation selon l'une quelconque des revendications 1 à 3, dans lequel :
a) le sujet est immunodéprimé ; ou :
b) le sujet est séropositif ou a un syndrome d'hyper-IgM ; et/ou :
c) le sujet a un nombre de lymphocytes T CD4⁺ inférieur à 200 cellules/µl de sang.

5. Composé pour utilisation selon l'une quelconque des revendications 1 à 4, dans lequel le composé 1, ou un sel pharmaceutiquement acceptable de celui-ci, est administré simultanément ou dans les 7 jours, avant et/ou après, que le sujet :
a) a subi une thérapie d'immunosuppression ; ou :
b) a subi une thérapie anti-TNF, une thérapie aux corticostéroïdes, ou une chimiothérapie ; ou :
c) a subi une opération de transplantation ; éventuellement où l'opération de transplantation est une transplantation d'organe ou une transplantation de moelle osseuse.

6. Composé destiné à être utilisé selon l'une quelconque des revendications 1 à 5, dans lequel le sujet est un humain et l'infection par Pneumocystis est provoquée par Pneumocystis jirovecii.

7. Composé pour utilisation selon l'une quelconque des revendications 1 à 6, dans lequel le composé est administré par voie intra-orale, par voie intraveineuse, par voie intramusculaire, par voie intradermique, par voie intra-artérielle, par voie sous-cutanée, par voie orale ou par inhalation.

8. Composé destiné à être utilisé dans un procédé *in vivo* d'inhibition de la réplication d'un Pneumocystis spp. comprenant la mise en contact du Pneumocystis spp. avec ledit composé en une quantité et pendant une durée suffisantes pour inhiber la réplication du Pneumocystis spp., où le composé est le composé 1 : 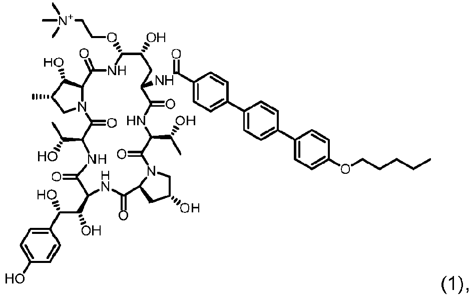 ou un sel pharmaceutiquement acceptable correspondant.

9. Composé pour utilisation selon la revendication 8, dans lequel le Pneumocystis spp. est Pneumocystis jirovecii.

10. Composé pour utilisation selon la revendication 1-9, le procédé comprenant l'administration au sujet de doses d'environ 150 mg à environ 800 mg d'un sel de composé 1, ou d'une forme neutre de celui-ci, une à trois fois par semaine au sujet pendant 2 à 8 semaines.

11. Composé pour utilisation selon l'une quelconque des revendications 1-10, où le composé est administré simultanément avec un deuxième agent antifongique ; facultativement dans lequel :
a) le deuxième agent antifongique est la clindamycine, le triméthoprime, le sulfaméthoxazole, le cotrimoxazole, l'atovaquone, la pentamidine, la primaquine, la pyriméthamine, ou un sel pharmaceutiquement acceptable correspondant ; ou :
b) le deuxième agent antifongique est choisi parmi les inhibiteurs de glucane synthase, les composés polyène, les composés azole et des sels pharmaceutiquement acceptables correspondants.

12. Composé pour utilisation selon l'une quelconque des revendications 1 à 11, le procédé comprenant l'administration une fois par semaine du composé 1 sous forme de sel ou neutre, le procédé comprenant
(a) l'administration par voie intraveineuse d'une première dose d'environ 400 mg du composé 1 sous forme de sel ou neutre,
(b) l'administration par voie intraveineuse d'une dose subséquente d'environ 200 mg ou environ 400 mg du composé 1 sous forme de sel ou neutre, et
(c) éventuellement la répétition de l'étape (b) pendant une à dix semaines.

13. Composé pour utilisation selon la revendication 12, dans lequel le procédé est constitué de
(a) l'administration par voie intraveineuse d'une première dose d'environ 400 mg du composé 1 sous forme de sel ou neutre,
(b) l'administration par voie intraveineuse d'une deuxième dose de 200 mg du composé 1 sous forme de sel ou neutre, et
(c) facultativement, l'administration par voie intraveineuse d'une troisième dose d'environ 200 mg du composé 1 sous forme de sel ou neutre,
dans lequel la première dose est administrée au jour 1, la deuxième dose est administrée au jour 8, et la troisième dose, si elle est administrée, est administrée au jour 15 ;
ou dans lequel le procédé est constitué de
(a) l'administration par voie intraveineuse d'une première dose d'environ 400 mg du composé 1 sous forme de sel ou neutre,
(b) l'administration par voie intraveineuse d'une deuxième dose d'environ 400 mg du composé 1 sous forme de sel ou neutre, et
(c) facultativement, l'administration par voie intraveineuse d'une troisième dose d'environ 400 mg du composé 1 sous forme de sel ou neutre,
dans lequel la première dose est administrée au jour 1, la deuxième dose est administrée au jour 8, et la troisième dose, si elle est administrée, est administrée au jour 15.

14. Composé pour utilisation selon l'une quelconque des revendications 1 à 13, dans lequel :
a) le composé 1 est administré sur une période de temps de 30 à 180 minutes ; et/ou ;
b) le composé 1 est administré sous la forme d'une composition pharmaceutique aqueuse ; facultativement, dans lequel la composition pharmaceutique a un pH de 4,0 à 8.

15. Composé pour utilisation selon l'une quelconque des revendications 1 à 14, dans lequel le sel du composé 1 est l'acétate du composé 1.
